(19) Europäisches Patentamt
European Patent Office
Office européen des brevets

(11) Publication number: 0 375 387 A2

(12) # EUROPEAN PATENT APPLICATION

(21) Application number: 89313329.8

(22) Date of filing: 20.12.89

(51) Int. Cl.5: C07C 233/13, A01N 37/18, C07C 323/42, C07C 235/28, C07C 233/18, C07C 233/25, C07C 233/15, C07C 233/66, C07D 307/52, C07D 333/20, A01N 43/08

(30) Priority: 23.12.88 JP 323620/88

(43) Date of publication of application: 27.06.90 Bulletin 90/26

(84) Designated Contracting States: DE FR GB IT NL

(71) Applicant: TOKUYAMA SODA KABUSHIKI KAISHA
1-1 Mikage-cho
Tokuyama-shi Yamaguchi-ken(JP)

(72) Inventor: Kato, Shozo
4-16-4-105, Azuma
Tsukuba-shi Ibaragi-ken(JP)
Inventor: Okamoto, Hidenori
1-13-7-202, Sengen
Tsukuba-shi Ibaragi-ken(JP)

(74) Representative: Myerscough, Philip Boyd et al
J.A.Kemp & Co. 14, South Square Gray's Inn
London, WC1R 5EU(GB)

(54) **Amide compounds.**

(57) An amide compound represented by the following general formula (I)

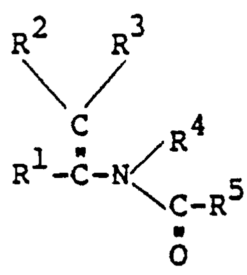

... (I)

wherein $R^1$ is a substituted or unsubstituted phenyl group, a substituted or unsubstituted furyl group, or a substituted or unsubstituted thienyl group; $R^2$ is a halogen atom; $R^3$ is a hydrogen atom, an alkyl group of 1-6 carbon atoms, or a halogen atom; $R^4$ and $R^5$ which may be the same or different, are a substituted or unsubstituted alkyl group of 1-12 carbon atoms, a substituted or unsubstituted alkenyl group of 2-12 carbon atoms, or a substituted or unsubstituted phenyl group: and an antimicrobial agent containing as an active ingredient the said amide compound.

EP 0 375 387 A2

# AMIDE COMPOUNDS

The present invention relates to a novel amide compound and an antimicrobial agent containing said amide compound as an active ingredient.

[Prior Art]

With respect to ethenylamide derivatives, there have conventionally been known a number of compounds as shown below.

(1) Zhurnai Organicheskoi Khimii, 18, 538 (1982)

This reference describes that the N-(2,2-di chloroethenyl)amide compounds represented by the general formula

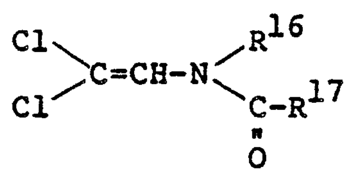

$R^{16}$ represents a methyl group, an ethyl group, an allyl group, a butyl group or a pentyl group, and $R^{17}$ represents a methyl group, an ethyl group, a propyl group or a butyl group) can be synthesized by a reaction of an N-(2,2,2-trichloroethylidene)amine derivative with an acid chloride in the presence of zinc, as shown by the following reaction formula.

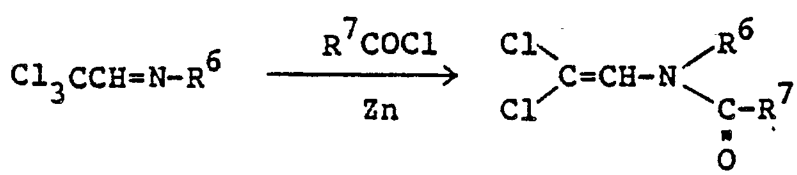


The ethenylamide derivative obtained by the above synthetic method, however, are restricted to N-(2,2-dichloroethenyl)amide compounds; and the reference makes no mention on those derivatives having a sub stituent at the 1-position of the ethenyl group. The paper makes no mention, either, on the research on specific application fields of said derivatives, such as biological activity and the like.

(2) Japanese Laid-Open Patent Publication No. 74670/83

This patent publication discloses anilide derivatives represented by the following general formula

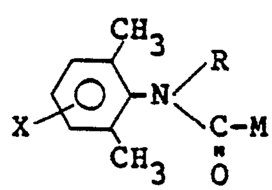

wherein X represents a lower alkyl group or a halogen atom; R represents a lower alkyl group, a cycloalkyl group, a methyl-substituted cycloalkyl group, a benzyl group, an α-phenethyl group, a lower alkyl-substituted α-phenethyl group or

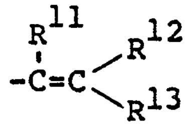

($R^{11}$ represents a hydrogen atom, a lower alkyl group, a phenyl group or a toluyl group; $R^{12}$ represents a hydrogen atom, a lower alkyl group or a halogen atom; $R^{13}$ represents a hydrogen atom or a lower alkyl group; $R^{11}$ and $R^{12}$ may each be a straight chain or branched chain alkylene group of 1-6 carbon atoms to bond to each other to form a ring); and M represents an imidazole group or a triazole group.

The patent publication describes that the above anilide derivatives have a herbicidal activity to various weeds, but makes no mention on other biological activities to animals and plants.

(3) Japanese Laid-Open Patent Publication No. 293956/86

This patent publication discloses chloroacetamide compounds represented by the following general formula

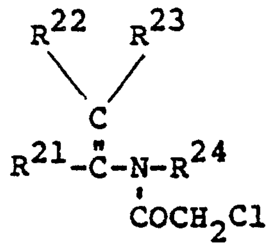

wherein $R^{21}$ is an unsubstituted or substituted aryl group or a heteroaryl group; $R^{22}$ and $R^{23}$ which may be the same or different, are a hydrogen atom or an alkyl group; $R^{22}$ and $R^{23}$ may bond to each other to form a ring; and $R^{24}$ is a hydrocarbon reside.

The patent publication describes that the above chloroacetamide compounds have a herbicidal activity to various weeds, but makes no mention on other biological activities.

The present inventors have long made research on the synthesis of a wide range of compounds having an excellent biological activity. Paying special attention to specific compounds having an enamine structure and, in particular, ethenylamide compounds, the present inventors have made extensive research on their synthesis and biological activity. As a result, it has been found that a group of novel specific amide compounds exhibit a strong antimicrobial activity to fungi and bacteria and accordingly are effective as an antimicrobial agent. This invention has been completed based on the finding.

According to the present invention, there are provided an amide compound represented by the following general formula (I)

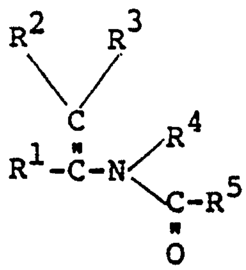

... (I)

wherein $R^1$ is a substituted or unsubstituted phenyl group, a substituted or unsubstituted furyl group, or a substituted or unsubstituted thienyl group; $R^2$ is a halogen atom; $R^3$ is a hydrogen atom, an alkyl group of 1-6 carbon atoms, or a halogen atom; $R^4$ and $R^5$ which may be the same or different, are a substituted or unsubstituted alkyl group of 1-12 carbon atoms, a substituted or unsubstituted alkenyl group of 2-12 carbon atoms, or a substituted or unsubstituted phenyl group; when $R^1$, $R^4$ and $R^5$ are each a substituted phenyl

group or when $R^1$ is a substituted furyl group or a substituted thienyl group, the substituent(s) each of the phenyl group, the furyl group and the thienyl group is (are) one to three members selected from the group consisting of halogen atoms, alkyl groups of 1-6 carbon atoms, alkoxy groups of 1-6 carbon atoms, alkylthio groups of 1-6 carbon atoms and halogenoalkyl groups of 1-4 carbon atoms; when $R^4$ and $R^5$ are each a substituted alkyl group or a substituted alkenyl group, the substituent(s) of each of the alkyl group and the alkenyl group is (are) one to three members selected from the group consisting of halogen atoms, alkoxy groups of 1-6 carbon atoms, alkylthio groups of 1-6 carbon atoms, a cyano group and a phenyl group (this phenyl group may be substituted by one to three members selected from the group consisting of halogen atoms, alkyl groups of 1-6 carbon atoms, alkoxy groups of 1-6 carbon atoms and alkylthio groups of 1-6 carbon atoms); and an antimicro bial agent containing said amide compound as an active ingredient.

The amide compound represented by the general formula (I) according to the present invention is a novel compound which, as far as the present inventors know, is not described in any literature. This novel amide compound has an excellent antimicrobial activity to a wide range of fungi and bacteria and accordingly is very useful as an active ingredient for use in antimicrobial agents.

The present invention is described in detail below.

The amide compound of the present invention is represented by the following general formula (I), as mentioned above.

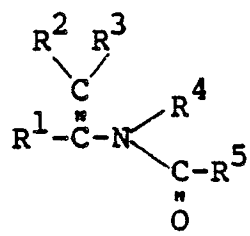 ... (I)

In the general formula (I), $R^1$ is a substituted or unsubstituted phenyl group, a substituted or unsubstituted furyl group or a substituted or unsubstituted thienyl group. When $R^1$ is a substituted phenyl group, a substituted furyl group or a substituted thienyl group, there are mentioned, as the substituents of these groups, particularly the following substituents in view of the antimicrobial activity and easiness of industrial production of intended compound. That is, as the substituents, there are mentioned halogen atoms, alkyl groups of 1-6 carbon atoms, alkoxy groups of 1-6 carbon atoms, alkylthio groups of 1-6 carbon atoms and halogenoalkyl groups of 1-4 carbon atoms. More speci fically, the halogen atoms include a fluorine atom, a chlorine atom, a bromine atom and an iodine atom; the alkyl groups include straight chain or branched chain alkyl groups such as methyl, ethyl, n-propyl, isopropyl, n-butyl, sec-butyl, isobutyl, t-butyl, n-pentyl, n-hexyl and the like; the alkoxy groups are preferably a methoxy group, an ethoxy group, a n-propoxy group, an isopropoxy group, a n-butoxy group, a n-pentyloxy group, a hexyloxy group, etc.; the alkylthio groups are preferably a methylthio group, an ethylthio group, a propylthio group, a butylthio group, a pentylthio group, a hexylthio group, etc.; and the halogenoalkyl groups are specifically a fluoromethyl group, a chloromethyl group, a trifluoromethyl group, a fluoroethyl group, etc.

When $R^1$ is a substituted phenyl group, a substituted furyl group or a substituted thienyl group, the number of the substituent(s) can be one or more, preferably one to three. When such $R^1$ has two or more substituents, the substituents may be the same or different.

When $R^1$ is a substituted phenyl group having substituent(s) as mentioned above, specific examples of the substituted phenyl group include alkylphenyl groups such as methylphenyl, dimethylphenyl, ethylphenyl, diethylphenyl, propylphenyl, dipropylphenyl, butylphenyl, pentylphenyl, hexylphenyl, methyl(ethyl)-phenyl, methyl(propyl)phenyl, ethyl(propyl)phenyl and the like; halophenyl groups such as fluorophenyl, difluorophenyl, chlorophenyl, dichlorophenyl, bromophenyl, iodophenyl, chloro(fluoro)phenyl and the like; alkoxyphenyl groups such as methoxyphenyl, dimethoxyphenyl, trimethoxyphenyl, ethoxyphenyl, diethoxyphenyl, propoxyphenyl, butoxyphenyl and the like; alkythiophenyl groups such as methylthiophenyl, dimethylthiophenyl, ethylthiophenyl, propylthiophenyl, butylthiophenyl, pentylthiophenyl, hexylthiophenyl and the like; haloalkylphenyl groups such as (chloromethyl) phenyl, (trifluoromethyl)phenyl and the like: and phenyl groups having different types of substituents, such as chloro(methyl)phenyl, fluoro(ethyl)phenyl, methyl-(methoxy)phenyl and the like.

Specific examples of the substituted furyl group and the substituted thienyl group include substituted furyl groups such as methylfuryl, dimethylfuryl, propylfuryl, chlorofuryl, bromofuryl, methoxyfuryl, ethoxyfuryl, propoxyfuryl, methylthiofuryl, ethylthiofuryl and the like; and substituted thienyl groups such as

methylthienyl, ethylthienyl, propylthienyl, butylthienyl, fluorothienyl, chlorothienyl, bromothienyl, iodothienyl, methoxythienyl, ethoxythienyl, propoxythienyl, methylthiothienyl, ethylthiothienyl and the like.

In the general formula (I), $R^2$ is a halogen atom. The halogen atom includes a fluorine atom, a chlorine atom, a bromine atom and an iodine atom. Of these, the chlorine atom and the bromine atom are particularly preferable in view of the biological activity, easiness of handling and easiness of industrial production of intended compound.

In the general formula (I), $R^3$ is a hydrogen atom, an alkyl group of 1-6 carbon atoms or a halogen atom. As examples of the alkyl group, there can be mentioned those specifically mentioned for the alkyl substituent of $R^1$; and there are preferred alkyl groups of 1-3 carbon atoms. As exmaples of the halogen atom, there can be mentioned those specifically mentioned for $R^2$; and there are preferred a chlorine atom and a bromine atom for the same reasons.

As mentioned above, in the compound (I) of the present invention, at least either of $R^2$ and $R^3$ is a halogen atom. This is believed to contribute to the antimicrobial activity exhibited by the compound of the present invention. When $R^2$ is a halogen atom and $R^3$ is a hydrogen atom, the resulting compound gives the highest antimicrobial activity. However, even when $R^2$ and $R^3$ are defined as above, the antimicrobial activity differs in some cases depending upon their combination with the types of $R^1$, $R^4$ and $R^5$; therefore, $R^2$ and $R^3$ need be appropriately selected in view of the types of $R^1$, $R^4$ and $R^5$.

Incidentally, the amide compound (I) of the present invention includes even a mixture wherein position isomers with respect to $R^2$ and $R^3$ coexist in various proportions. That is, $R^2$ and $R^3$ may take any of a cis-position and a trans-position.

In the general formula (I), $R^4$ and $R^5$ which may be the same or different, are a substituted or unsubstituted alkyl group of 1-12 carbon atoms, a substituted or unsubstituted alkenyl group of 2-12 carbon atoms, or a substituted or unsubstituted phenyl group.

As the alkyl group, there are preferred alkyl groups of 1-6 carbon atoms; and as the alkenyl group, there are preferred alkenyl groups of 2-6 carbon atoms.

Of the alkyl groups of 1-6 carbon atoms, preferable are those specifically mentioned for the alkyl substituent of $R^1$; and of the alkyl groups of 7-12 carbon atoms, preferably are a heptyl group, an octyl group, a nonyl group, a decyl group, etc.

In the above alkyl groups, one or more, preferably one to two of the hydrogen atoms constituting each alkyl group may be each substituted by a substituent. As the substituent, the followings are particularly preferable in view of the antimicrobial activity and easiness of industrial production of intended compound. That is, there are mentioned, for example, halogen atoms, alkoxy groups of 1-6 carbon atoms; alkylthio groups of 1-6 carbon atoms; a cyano group; and a phenyl group which may be substituted by one to three members selected from the group consisting of alkyl groups of 1-6 carbon atoms, alkoxy groups of 1-6 carbon atoms, alkylthio groups of 1-6 carbon atoms and halogen atoms.

The halogen atoms include a fluorine atom, a chlorine atom, a bromine atom and an iodine atom. The alkoxy groups are preferably a methoxy group, an ethoxy group, a propoxy group, a butoxy group, a pentyloxy group, a hexyloxy group, etc. The alkylthio groups are preferably a methylthio group, an ethylthio group, a propylthio group, a butylthio group, a pentylthio group, a hexylthio group, etc.

As examples of the substituted phenyl groups as the substituents in $R^4$ and $R^5$ when $R^4$ and $R^5$ are each a substituted alkyl group, there are preferred those substituted phenyl groups specifically mentioned for $R^1$.

When $R^4$ and $R^5$ are a substituted alkyl group, particularly preferably examples of the substituted alkyl group are straight chain or branched chain haloalkyl groups such as fluoromethyl, trifluoromethyl, chloromethyl, trichloromethyl, chloroethyl, bromoethyl, fluoropropyl, chloropropyl, chlorobutyl, bromopentyl, chlorohexyl, and the like; straight chain or branched chain alkoxyalkyl groups such as methoxymethyl, methoxyethyl, dimethoxyethyl, methoxypropyl, methoxybutyl, methoxypentyl, methoxyhexyl, ethoxymethyl, ethoxyethyl, diethoxyethyl, ethoxypropyl, diethoxypropyl, ethoxybutyl, propoxymethyl, propoxyethyl, propoxypropyl, propoxybutyl, butoxymethyl, butoxyethyl, butoxypropyl, butoxybutyl, pentoxyethyl and the like; alkylthioalkyl groups such as methylthiomethyl, methylthioethyl, methylthiopropyl, ethylthiomethyl, ethylthioethyl, ethylthiobutyl, propylthioethyl and the like; cyanoalkyl groups such as cyanoethyl, cyanopropyl, cyanobutyl and the like; and phenyl alkyl groups such as phenylmethyl, phenylethyl, phenyl propyl, (methylphenyl)methyl, (ethylthiophenyl)methyl, (chlorophenyl)propyl and the like.

When $R^4$ and $R^5$ are an alkenyl group, specific exmaples of the alkenyl group include various alkenyl groups of position isomerism, such as ethenyl, propenyl, butenyl, pentenyl, hexenyl, octenyl and the like.

In the above alkenyl groups, one or more, preferably one to two of the hydrogen atoms constituting each alkenyl group may each be substituted by a substituent. As the substituent, the following are particularly preferable in view of the antimicrobial activity and easiness of industrial production of intended

compound. That is, there are mentioned, for example, halogen atoms such as fluorine atom, chlorine atom, bromine atom, iodine atom and the like; alkoxy groups such as methoxy, ethoxy, propoxy, butoxy and the like; alkylthio groups such as methylthio, ethylthio, propylthio, butylthio and the like; and a phenyl group which may be substituted by one to three members selected from the group consisting of halogen atoms, alkyl groups of 1-6 carbon atoms, alkoxy groups of 1-6 carbon atoms and alkylthio groups of 1-6 carbon atoms.

In the general formula (I), when $R^4$ and $R^5$ are a substituted phenyl group, specific examples of the substituted phenyl group are preferably those specifically mentioned for $R^1$.

In the general formula (I), the structure of $R^4$ has a large effect on the antimicrobial activity of the compound (I) to fungi and bacteria. When $R^4$ is a branched alkyl group or a branched aralkyl group, the compound (I) has a particularly high antimicrobial activity. As the alkyl group having side chain(s), there is particularly preferred such an alkyl group whose carbon atom bnding to the nitrogen atom of the compound (I)(i.e. the carbon atom at the $\alpha$-position) has side chain(s).

In the compound of the general formula (I), various position isomers exist in many cases. These compounds can be used in the present invention irrespective of the position isomerism. For exmaple, the methylphenyl group can be any of o-methylphenyl, m-methylphenyl and p-methylphenyl; and the butyl group can be any of n-butyl, sec-butyl and t-butyl.

Further in the compound of the general formula (I), the substituents are not restricted to those specifically mentioned above, and any substituent can be appropriately selected as necessary as long as it meets the definition of the general formula (I).

As described above, the amide compound represented by the general formula (I) is a novel compound and has a high antimicrobial activity to microbes such as fungi, bacteria and the like. Accordingly, the amide compound of the present invention can be used as an antimicrobial agent. The biological activity of the amide compound of the present invention has been newly made known by the present invention.

The process for producing the present compound represented by the general formula (I) has no particular restriction. A typical process for producing the compound is as follows.

A Schiff base compound represented by the general formula (II)

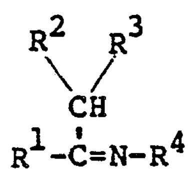

... (II)

[$R^1$, $R^2$, $R^3$ and $R^4$ have the same definitions as in the general formula (I)] is reacted with a carboxylic acid derivative represented by the general formula (III)

O
||
$R^5$- C -X (III)

[$R^5$ has the same definition as in the general formula (I), and X represents a halogen atom or

O
||
-O- C -$R^5$], whereby an amide compound represented by the general formula (I) can be obtained.

The Schiff base compound represented by the general formula (II), used as a material in the above reaction, can be produced by various processes. It is ordinarily obtained by, as shown in the following reaction formula, a dehydrocondensation reaction between a halogen atom-containing ketone compound and an amine compound, or a halogenation reaction of a Schiff base compound.

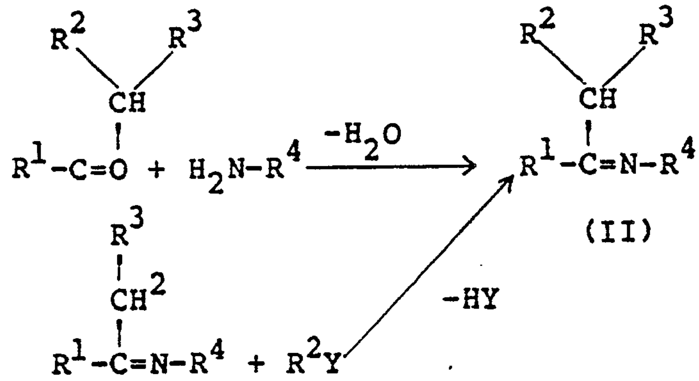


[Y is a halogen atom or a

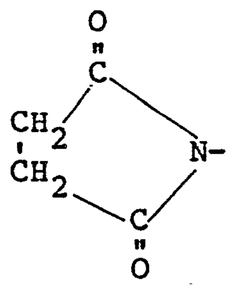

group; and

$R^1$, $R^2$, $R^3$ and $R^4$ have the same definition as in the general formula (I)].

The Schiff base compound represented by the general formula (II), used as a material in the present invention, is not necessarily an isolated and purified product. That is, the halogen atom-containing ketone compound is reacted with the amine compound, or the Schiff base compound is subjected to a halogenation reaction, and the reaction mixture itself can be reacted with a carboxylic acid derivative represented by the general formula (III).

In the reaction of the Schiff base compound represented by the general formula (II) with the carboxylic acid derivative represented by the general formula (III), the molar ratio of the two compounds can be appropriately determined so as to meet the requirements, but they are ordinarily used in an equimolar ratio.

In said reaction, there is formed, as a by-productg, an acidic compound such as hydrogen halide or the like. Therefore, it is ordinarily preferable to use in the reaction an acid scavenger for hydrogen halide or the like. The acid scavenger is not particularly restricted and can be a known acid scavenger. As the preferable scavenger used generally, there can be mentioned bases such as triethylamine, tripropylamine, pyridine, sodium alcoholate, sodium hydrogencarbonate, sodium carbonate, potassium carbonate and the like.

In the above reaction, it is generally preferable to use an organic solvent. As preferable examples of the organic solvent, there can be mentioned benzene, toluene, xylene, hexane, petroleum ether, carbon tetrachloride, chloroform, methylene chloride, ethyl ether, dioxane, tetrahydrofuran, acetone, methyl ethyl ketone, acetonitrile, N,N-dimethylformamide, hexamethylphosphoramide, dimethyl sulfoxide, etc.

When there is used, as the organic solvent, a basic amide type polar solvent such as N,N-dimethylformamide, N,N-dimethylacetamide, hexamethylphosphoramide or the like, the above reaction proceeds easily in many cases even without using the acid scavenger for hydrogen halide or the like, enabling the production of an intended amide compound at a high yield. Therefore, the use of such a solvent is very preferable.

In the above reaction, the addition order of the materials has no particular restriction. But generally, the Schiff base compound represented by the general formula (II) is dissolved in a solvent and then the carboxylic acid derivative represented by the general formula (III) is added thereto with stirring.

In the above reaction, the reaction temperature can be selected in a wide range generally between -20 °C and 150 °C, preferably between -10 °C and 120 °C. The reaction time differs by the materials and reaction temperature used, but can be selected to be ordinarily 5 minutes to 10 days, preferably 1-50 hours. Also, it is preferable to effect stirring during the reaction.

The method for isolating the intended product, i.e. the amide compound represented by the general formula (I) from the reaction mixture and purifying it is not particularly restricted, and there can be used

various methods. For example, after the reaction, water is added to the reaction mixture, and the resulting residue is extracted with benzene, ether, chloroform or the like. The extract is dried with a drying agent such as sodium sulfate, calcium chloride or the like. Then, the solvent is removed by distillation and the residue is subjected to vacuum distillation to obtain an intended compound. Besides vacuum distillation, there can be used chromatography, recrystallization, etc. for purification.

The properties of the amide compound (I) of the present invention vary slightly depending upon the types of $R^1$, $R^2$, $R^3$, $R^4$ and $R^5$ in the general formula (I) and the extent of the purification. However, the amide compound (I) is generally a colorless to blackish brown viscous liquid or solid at normal temperature and normal pressure. Further, the compound (I) of the present invention is soluble in ordinary organic solvents such as benzene, ether, alcohol, chloroform, acetonirile, dimethylformamide, dimethyl sulfoxide and the like, but is difficulty soluble in water.

When the reaction is effected using, as a reaction solvent, an amide type polar solvent such as N,N-dimethylformamide or the like, low-boiling substances are removed by distillation after the completion of the reaction, and then the residue is subjected simply to vacuum distillation or recrystallization, whereby an intended compound can be obtained easily. Therefore, the use of such an amide type polar solvent is advantageous.

The structure of the present compound represented by the general formula (I) can be confirmed by the following means.

(i) By measuring the infrared (IR) absorption spectrum, there can be observed an absorption due to CH bond, at about 3200-2800 $cm^{-1}$, and a strong absorption due to carbonyl group of amide, at about 1700-1640 $cm^{-1}$.

(ii) By measuring the mass spectrum (ms) and determining an atomic group corresponding to each spectral peak (generally the number represented by m/e where m is an ion molecular weight and e is the ion charge number), there can be known the molecular weight of test compound and the bonding mode of each atomic group in the compound molecule. That is, when the test compound represented by the following general formula (I)

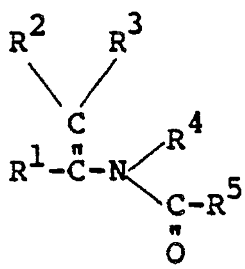

... (I)

is measured for mass spectrum, there is generally observed a molecular ion peaks (hereinafter bbreviated to $M^{\oplus}$ or $M^{+}$ 1), whereby the molecular weight of the test compound can be determined. Further in that case, there are also observed characteristic peaks corresponding to $M^{\oplus}$-$R^2$ (halogen atom), $M^{\oplus}$-$R^4$ and $M^{\oplus}$-$\overset{O}{\overset{\|}{C}}$-$R^5$, whereby the bonding mode of each atomic group in the molecule can be known.

(iii) By measuring the $^1$H-NMR spectrum, there can be known the bonding modes of hydrogen atoms present in the present compound represented by the general formula (I).

As a typical example of the $^1$H-NMR ($\delta$, ppm: measured in deuterated chloroform (solvent) using tetramethylsilane as a reference) of the compound represented by the general formula (I), there are shown below the analytical result of the following compound.

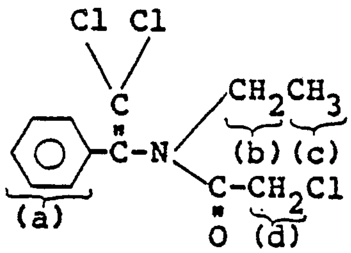

That is, there are observed a singlet of five protons due to phenyl group (a) at 7.40 ppm, a singlet of two protons due to methylene group (d) at 4.21 ppm, multiplets of two protons due to methylene (b) at 4.05-3.58 ppm and 3.27-2.68 ppm, and a triplet of three protons due to methyl group (c) at 1.21 ppm.

(iv) By determining the weight % of each of C, H, N and halogen (also S when S is contained) by elemental analysis and subtracting the sum of each weight % obtained, from 100, there can be calculated the weight % of oxygen, whereby the composition of the present compound can be determined.

The amide compound represented by the general formula (I) according to the present invention has an excellent antimicrobial activity. This antimicrobial activity is surprising and unexpected in view of the fact that, as shown in Comparative Examples 1 and 2 (described later), compounds having a structure similar to that of the present amide compound of the general formula (I) have no antimicrobial activity.

Said antimicrobial activity is seen in most of the compounds represented by the general formula (I), but varies slightly depending upon the types of $R^1$, $R^2$, $R^3$, $R^4$ and $R^5$ of the general formula (I).

Of the present amide compounds represented by the general formula (I), the amide compounds represented by the following general formula (I-a) exhibit the most striking antimicrobial activity to fungi, bacteria, etc.

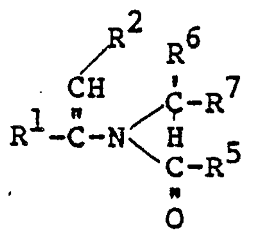

... (I-a)

[$R^1$, $R^2$ and $R^5$ have the same definitions as in the general formula (I); $R^6$ is an alkyl group of 1-6 carbon atoms; $R^7$ is an alkyl group of 1-6 carbon atoms or a phenyl group.]

Also of the amide compounds represented by the general formula (I-a), those having, as $R^5$, an alkyl group of 1-6 carbon atoms, preferably 1-3 carbon atoms, a haloalkyl group of 1-3 carbon atoms or an alkoxyalkyl group of 1-6 carbon atoms and, as $R^6$, a methyl group or an ethyl group, are particularly preferable because they have an antimicrobial activity to a wide range of fungi and bacteria.

The amide compound represented by the general formula (I) according to the present invention can be widely used to, for example, various pathogenic fungi and bacteria belonging to Basidiomycetes, Phycomycetes, Ascomycetes, imperfect fungi, bacteria, etc. and are particularly advantageous when used as an antimicrobial agent for agriculture and horticulture or as an antimicrobial agent for industries. Typical examples of the bacteria and fungi to which the amide compound of the present invention is effective as an antimicrobial agent, are shown below. However, such bacteria and fungi are not restricted to them.

Bacteria

Bacillus cereus
Bacillus megaterium
Bacillus subtilis
Bacillus subtillis var niger
Cladosporium herbarum
Escherichia coil
Klebsiella pneurnoniae
Leuconoster mesenteri
Pseudomonas aeruginosa
Proteus mirabiris
Proteus rettgeri
Proteus vulgaris
Rosellia necatrix
Staphylococcus aureus
Staphylococcus espidermidis
Streptococcus faecalis
Staphylococcus flova

Serpula lacrymans
Salomonella typhy

Fungi

Aspergillus orysae
Aspergillus niger
Aspergillus terreus
Cochliobolus miyabeanus
Fusarium monilifome
Fusarium oxysporum
Penicillium citrnum
Penicillium chrysoqenum
Phizopus nigricans
Phizopus stolonifer
Trichophyton rubrum

In preparing an antimicrobial agent containing the present amide compound of the general formula (I) as an active ingredient, the form of the antimicrobial agent is not particularly restricted. Forms used for conventionally known antimicrobial agents can be employed.

The amide compound (I) can be used in various desired forms, for example, a wettable powder, granules, an emulsion, a water solution, a flowable, an oil, a powder, tablets, an aerosol, a fumigant, a slow-release formulation and an immobilized formulation, by mixing with an inactive solid carrier, a liquid carrier, an emulsifying or dispersing agent, etc.

The antimicrobial agent of the present invention is used in a liquid or solid state where the amide compound (I) of the present invention is present as an admix with adjuvants such as carrier, diluent, spreading agent, conditioning agent and the like. At that time, the use of a surfactant is effective to obtain better dispersibility in water or oil. The surfactant refers herein to a wetting agent, a dispersing agent, a suspending agent, an emulsifying agent, etc. These surfactants can be any of anionic type, cationic type and nonionic type.

As the preferable wetting agent, there can be mentioned alkylbenzenesulfonates, alkylnaphthalenesulfonates, sulfonated fatty alcohols, esters of sodium sulfosuccinate, esters of sodium isothionate, sulfated or sulfonated fatty acid esters, petroleum sulfonates, polyoxyethylene alkylphenyl ether sulfonates, sodium alkyl sulfates, etc.

As the preferable dispersant, there can be mentioned methyl cellulose, polyvinyl alcohol, sodium ligninsulfonate, polymeric alkylnaphthalenesulfonates, sodium tripolyphosphate, polymeric alkyl sodium naphthalene sulfonates, etc.

The form of the antimicrobial agent of the present invention can be various, as described below.

The wettable powder and the granules are water-dispersible and contain at least one active ingredient, an inactive solid carrier, at least one surfactant, etc. As the inactive solid carrier, there are generally used fine powders of natural and synthetic minerals. For example, there are used clays of pyrophyllite type, kaolinite type, etc.; talc; heavy calcium carbonate; diatomaceous earth; and silica gel. The wettable powder ordinarily has a comosition comprising 0.1-90 parts, preferably 1-50 parts of an active ingredient, 0.25-25 parts, preferably 1-15 parts of a wetting agent, 0.25-25 parts, preferably 1-15 parts of a dispersant and 5-95 parts, preferably 5-50 parts of an inactive solid carrier. (In the above, parts refer to parts by weight. The same applies hereinafter.) As necessary, a corrosion inhibitor, an antifoaming agent and an auxiliary agent can be added.

The emulsion is generally a solution obtained by dissolving an active ingredient and a surfactant in a solvent. The solvents suitable for the active ingredient of the present invention are dimethylformamide, dimethyl sulfoxide, N-methylpyrrolidone, hydrocarbons, lower alcohols, ethers, ketones, aromatic compounds (e.g. toluene, xylene, methylnaphthalene), etc. The emulsion generally has a composition comprising 0.1-95 parts, preferably 10-60 parts of an active ingredient, 0.25-50 parts, preferably 1-25 parts of a surfactant and 4-90 parts of a solvent.

The water solution is a transparent solution obtained by dissolving an active ingredient in water and a water-soluble solvent. In order to prepare a water solution, the active ingredient is preferably water-soluble. Even when the active ingredient is water-insoluble, it is possible to dissolve the active ingredient in water, by adding a solubilizing agent. As the solubilizing agent, there can be mentioned alkalis such as sodium hydroxide, ammonia, amines and the like; acids such as hydrochloric acid, sulfuric acid and the like;

surfactants; chelating agents; and so forth. Also, it is often preferable to add an auxiliary solvent such as denatured alcohol, ethylene glycol, polyethylene glycol, cellosolve or the like, in order to increase the stability of the water solution.

The oil is obtained by dissolving an active ingredient of no or low water solubility in an organic solvent. Preferable as the organic solvent are those specifically mentioned with respect to the solvent for the emulsion. In many cases, there are also used preferably those surfactants mentioned above.

The powder is obtained by mixing an active ingredient with a mineral powder (e.g. clay, talc) and other additives.

The flowable is a suspension agent obtained by making a water-insoluble active ingredient into a fine powder and dispersing the fine powder in water with the aid of a dispersing agent or the like.

The slow-release formulation can retain the effect of active ingredient for a longer period by the controlled release of active ingredient and is prepared, for example, by sealing an active ingredient in microcapsules, or by allowing the active ingredient to be held by a porous substance such as zeolite, silica gel or the like, or by including the active ingredient in an inclusion compound such as cyclodextrine or the like.

The immobilized formulation is prepared by immobilizing an active ingredient on the surface of an organic or inorganic insoluble carrier via covalent bond.

The fumigant contains a heat-generating agent (e.g. nitric acid salt, nitrous acid salt, guanidine salt, potassium chlorate) and a heat generation controlling agent (e.g. alkali metal salt, potassium nitrate) for vaporizing the active ingredient used therein.

In using the present amide compound represented by the general formula (I) in the form of an antimicrobial agent, the amount of the compound applied can be selected in a wide range. For example, when plants are treated, the amount of the compound applied can be generally 1-0.0001 % by weight, preferably 0.5-0.001 % by weight based on the weight of plant. When seeds are treated, the amount of the present compound applied can be generally 0.001-50 g, preferably 0.01-10 g per 1 kg of seed. In treating soils, the amount of the present compound applied can be 0.00001-0.1 % by weight, preferably 0.0001-0.02 % by weight based on the weight of soil.

The compound represented by the general formula (I) according to the present invention can be used in admixture with other antimicrobial agents, insecticides, herbicides, fertilizers, soil improvers, etc.

The present invention is described more specifically below by way of Examples. However, the present invention is in no way restricted to these Examples.

## EXAMPLE 1

In an eggplant-shape flask were placed N-(1-phenyl-2,2-dichloroethylidene)ethylamine (2.00 g, 0.0092 moles) and DMF (N,N-dimethylforamide) (20 ml). Thereto was dropwise added chloroacetyl chloride (1.32 g) at room temperature with stirring. The mixture was stirred for 2 days at room temperature. The reaction mixture was washed with water and then the organic layer was extracted with ether. The extract was dried with sodium sulfate. The low-boiling materials were removed. The resulting reddish brown liquid was purified by column chromatography to obtain light green crystals (2.05 g).

The results of instrumental analysis are shown below. Melting point: 47-49 °C

$^1$H-NMR spectrum (δ, ppm: measured in $CDCl_3$ using tetramethylsilane as a reference)

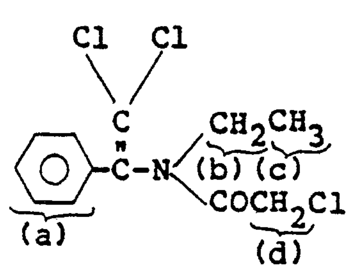

(a) 7.40 ppm (S, 5H)
(b) 4.05-3.58, 3.27-2.68 ppm (m, 2H)
(c) 1.12 ppm (t, 3H)
(d) 4.21 ppm (S, 2H)

Mass spectrum
m/e = 294, 292 ($M^{\oplus}$ +1), 256 (100, ($M^{\oplus}$ -Cl)
IR characteristic absorption

1675 $cm^{-1}$ (C=0)
Elemental analysis
Found: C 49.53 %, H 4.15 %, N 4.90 %
Calcualted as $C_{12}H_{12}NCl_3O$ (292.60):
C 49.26 %, H 4.13 %, N 4.79 %

From the above results, it was confirmed that the isolated product was N-(1-phenyl-2,2-dichloroethenyl)-N-chloroaceto-ethylamide. The yield was 76 %. This compound is designated as compound No. 1.

## EXAMPLE 2

In an eggplant-shape flask were placed N-(1-phenyl-2,2-dichloroethylidene)-ethylamide (1.80 g, 0.0083 mole), triethylamine (1.10 g, 0.011 mole) and chloroform (20 ml). Thereto was dropwise added acetyl chloride (1.20 g, 0.015 mole) with stirring under ice cooling. The mixture was stirred for a while at room temperature and then stirred for 2 hours in an oil bath of 50 °C. The resulting mixture was washed with water. The organic layer was extracted with chloroform. The extract was dried with sodium sulfate. The low-boiling materials were removed. The resulting liquid was purified by column chromatography to obtain a yellow solid (1.45 g).

The results of instrumental analysis are shown below.
Melting point: 45-47 °C
$^1$H-NMR spectrum (δ ppm: measured in $CDCl_3$ using tetramethylsilane as a reference)

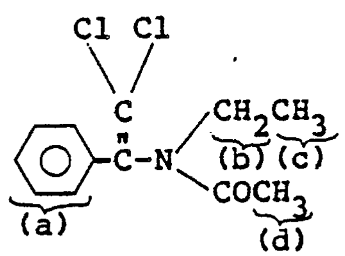

(a) 7.36 ppm (S, 5H)
(b) 2.20 ppm (S. 3H)
(c) 4.02-3.57,
3.00-2.55(m, 2H)
(d) 1.06 ppm (t, 3H)

Mass spectrum
m/e = 260, 258 ($M^{\oplus}$+1), 220 (100, ($M^{\oplus}$ -Cl)
IR characteristic absorption
1660 $cm^{-1}$ (C=0)
Elemental analysis
Found: C 56.05 %, H 5.07 %, N 5.12 %
Calcualted as $C_{12}H_{12}NCl_3O$ (258.14):
C 55.83 %, H 5.08 %, N 5.43 %

From the above results, it was confirmed that the isolated product was N-(1-phenyl-2,2-dichloroethenyl)-N-acetoethylamide. The yield was 67 %. This compound is designated as compound No. 2.

## EXAMPLE 3

In an eggplant-shape flask were placed N-(1-phenyl-2-chloroethylidene)-isopropylamine (2.63 g, 0.012 mole) and DMF (20 ml). Thereto was dropwise added chloroacetyl chloride (1.53 g, 0.013 mole) with stirring at room temperature. The mixture was stirred overnight at room temperature and then stirred for 2 hours in an oil bath of 50 °C. The resulting mixture was washed with water, and the organic layer was extracted with ether. The extract was dried with sodium sulfate. The low-boiling materials were removed. The resulting reddish brown liquid was purified by column chromatography (benzene/acetone = 1/30) to obtain pale yellow crystals (0.40 g).

The results of instrumental analysis are shown below.
Melting point: 56-56.5 °C
$^1$H-NMR spectrum (δ, ppm: measured in $CDCl_3$ using tetramethylsilane as a reference)

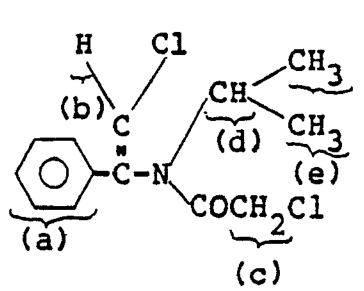

(a), (b) 7.46-7.05 ppm (m, 6H)
(c) 4.43 ppm (S, 2H)
(d) 3.48-3.10 ppm
(e) 1.12 ppm (d, 6H)

Mass spectrum
m/e = 273, 271 ($M^{\oplus}$), 222 (100, $M^{\oplus}$ $-CH_2Cl$)
IR characteristic absorption
1560 $cm^{-1}$ (C=0)
Elemental analysis
Found: C 57.30 %, H 5.59 %, N 5.09 %
Calcualted as $C_{13}H_{15}NCl_2O$ (272.17): C 57.37 %, H 5.56 %, N 5.15 %

From the above results, it was confirmed that the isolated product was N-(1-phenyl-2-chloro-ethenyl)-N-chloroacetoisopropylamide. The yield was 13 %. This compound is designated as compound No. 3.

EXAMPLE 4

In an eggplant-shape flask were placed N-chlorosuccinimide (3.30 g, 0.025 mole) and carbon tetrachloride (30 ml). Thereto was dropwise added a solution of N-[1-(4-methylphenyl)-propylidene]-2′-methoxyethylamine (5.20 g, 0.024 mole) dissolved in carbon tetrachloride (10 ml), with stirring under ice cooling. The mixture was stirred for 2 hours at room temperature. The solid was removed by filtration. To the filtrate was dropwise added a solution of chloroacetyl chloride (2.72 g, 0.024 mole) dissolved in carbon tetrachloride (5 ml), with stirring at room temperature. The mixture was stirred for a while at room temperature and then stirred for 1 hour in an oil bath of 50 °C. The low-boiling materials were removed. The resulting liquid was purified by column chromatography to obtain a pale yellow viscous liquid (4.30 g).

The results of instrumental analysis are shown below.

$^1$H-NMR spectrum (δ, ppm: measured in $CDCl_3$ using tetramethylsilane as a reference)

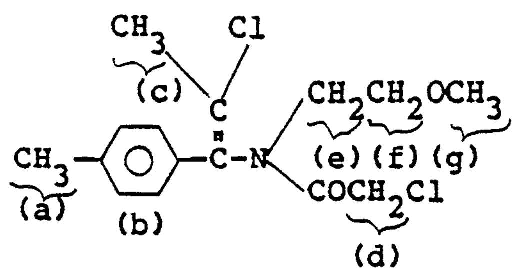

(a) 2.36 ppm (S, 3H)
(b) 7.18 ppm (S, 4H)
(c) 2.29 ppm (S, 3H)
(d) 4.36 ppm (S, 2H)
(e) 4.01-3.75, 3.13-2.87 ppm (m, 2H)
(f) 3.46 ppm (t, 2H)
(g) 3.22 ppm (S, 3H) Mass spectrum

m/e = 318, 316 ($M^{\oplus}$ +1), 280 ($M^{\oplus}$-Cl)
IR characteristic absorption
1680 $cm^{-1}$ (C=0)
Elemental analysis

Found: C 57.08 %, H 6.10 %, N 4.53 %

Calcualted as $C_{15}H_{19}NCl_2O_2$ (316.22): C 56.97 %, H 6.06 %, N 4.43 %

From the above results, it was confirmed that the isolated product was N-[1-(p-methyl-phenyl)-2-chloro-1-propenyl)-N-chloroaceto-2′-methoxyethylamide. The yield was 56 %. This compound is designated as compound No. 4.

## EXAMPLE 5

Various amide compounds having the following structure were synthesized in the same manner as in Examples 1-4. There are shown in Table 1 the compound Nos., structures, appearances, IR characteristics ab sorptions, mass spectra (the highest values of observed m/e values, i.e. $M^{\oplus}+1$, $M^{\oplus}$ or $M^{\oplus}$ - halogen) and elemental analyses of the compounds.

$R^1$, $R^2$, $R^3$, $R^4$ and $R^5$ in Table 1 correspond to $R^1$, $R^2$, $R^3$, $R^4$ and $R^5$ in the following formula, respectively.

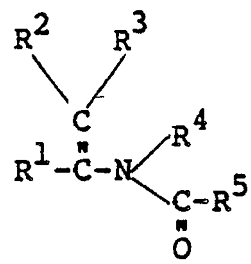

Incidentally, the amide compounds in Table 1 include even a mixture of position isomers with respect to $R^2$ and $R^3$.

The yields of the amide compounds synthesized in this Example varied from 13 % to 78 % depending upon the types of substituents ($R^1$, $R^2$, $R^3$, $R^4$ and $R^5$ and reaction solvent as well as upon the level of reaction temperature.

TABLE 1

| Compound No. | $R^1$ | $R^2$ | $R^3$ | $R^4$ | $R^5$ |
|---|---|---|---|---|---|
| 5 | 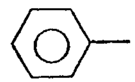 | Cl, | H | $-CH_3$ | 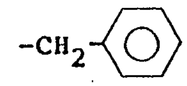 |
| 6 | 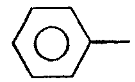 | Cl, | H | 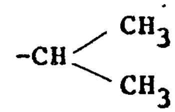 | $-CH_3$ |
| 7 | 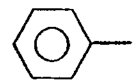 | Cl, | H | 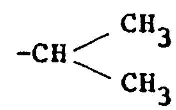 | $-C_2H_5$ |
| 8 | 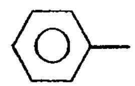 | Cl, | H | 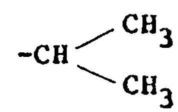 | 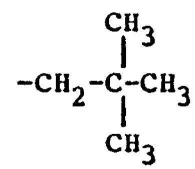 |
| 9 | 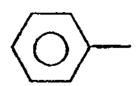 | Cl, | H | 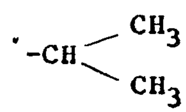 | $-(CH_2)_3-Cl$ |
| 10 | 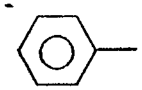 | Cl, | H | 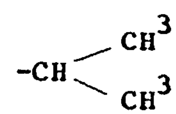 | $-CH=CHCH_3$ |

(continued)

TABLE 1 (continued)

| Compound No. | Appearance | IR($cm^{-1}$) (C=O) | Mass (m/e) | Elemental analysis (%) (Figures in parenthesis are calculated figures) |
|---|---|---|---|---|
| 5 | yellow solid | 1670 | 287<br>285 | C 71.38 H 5.80 N 4.71<br>(71.45) (5.64) (4.90) |
| 6 | yellow solid | 1660 | 237 | C 65.00 H 6.28 N 5.50<br>(65.68) (6.79) (5.89) |
| 7 | yellow viscous liquid | 1650 | 254<br>252 | C 66.47 H 7.50 N 5.22<br>(66.79) (7.21) (5.57) |
| 8 | yellowish brown viscous liquid | 1690 | 294 | C 69.11 H 8.50 N 4.39<br>(64.49) (8.23) (4.77) |
| 9 | pale yellow viscous liquid | 1740 | 264 | C 59.80 H 6.77 N 4.38<br>(60.01) (6.38) (4.67) |
| 10 | yellow viscous liquid | 1690 | 265<br>263 | C 68.41 H 6.93 N 5.35<br>(68.30) (6.88) (5.31) |

(continued)

TABLE 1 (continued)

| Compound No. | $R^1$ | $R^2$ $R^3$ | $R^4$ | $R^5$ |
|---|---|---|---|---|
| 11 | 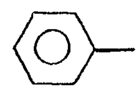 | Cl, H | 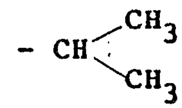 | 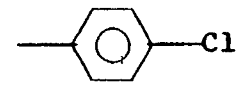 |
| 12 | 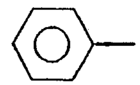 | Cl, H | 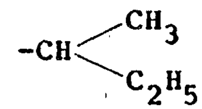 | 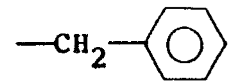 |
| 13 | 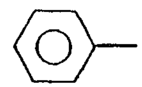 | Cl, H | 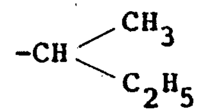 | $-C_2H_5$ |
| 14 | 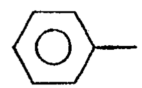 | Cl, H | $-(CH_2)_2-OCH_3$ | $-CH_2Cl$ |
| 15 | 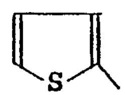 | Cl, H | $-(CH_2)_3-OC_2H_5$ | $-CH_2CH_2SCH_3$ |
| 16 | 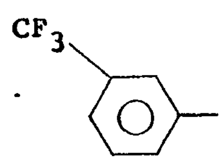 | Cl, H | 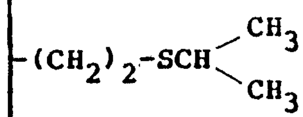 | $-CH_2CH_2Cl$ |

(continued)

TABLE 1 (continued)

| Compound No. | Appearance | $IR(cm^{-1})$ (C=0) | Mass (m/e) | Elemental analysis (%) (Figures in parenthesis are calculated figures) |
|---|---|---|---|---|
| 11 | yellow solid | 1650 | 335<br>333 | C 64.77 H 5.30 N 3.89<br>(64.68) (5.13) (4.19) |
| 12 | yellow viscous liquid | 1660 | 327 | C 73.38 H 6.45 N 4.67<br>(73.27) (6.77) (4.27) |
| 13 | yellow viscous liquid | 1660 | 265 | C 67.85 H 7.79 N 5.02<br>(71.45) (7.58) (5.27) |
| 14 | yellow viscous liquid | 1690 | 290<br>288 | C 54.36 H 5.47 N 4.65<br>(54.18) (5.25) (4.86) |
| 15 | brown viscous liquid | 1680 | 349<br>347 | C 51.61 H 6.40 N 3.98<br>(51.78) (6.37) (4.03) |
| 16 | yellow viscous liquid | 1670 | 415<br>413 | C 49.37 H 4.80 N 3.33<br>(49.28) (4.87) (3.38) |

(continued)

TABLE 1 (continued)

| Compound No. | $R^1$ | $R^2$ $R^3$ | $R^4$ | $R^5$ |
|---|---|---|---|---|
| 17 | 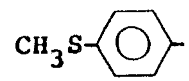 | Cl, H | $-CH_2CH_2CN$ | 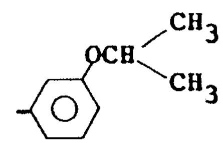 |
| 18 | 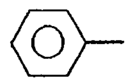 | Cl, H | 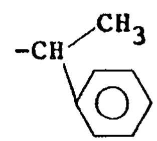 | $-CH_3$ |
| 19 | 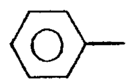 | Cl, H | 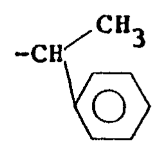 | $-C_2H_5$ |
| 20 | 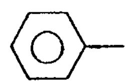 | Cl, H | 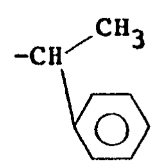 | $-CH_2Cl$ |
| 21 | 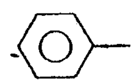 | Cl, H | 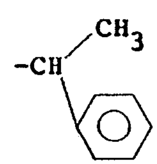 | $-CH_2OCH_3$ |

(continued)

TABLE 1 (continued)

| Compound No. | Appearance | IR($cm^{-1}$) (C=0) | Mass (m/e) | Elemental analysis (%) (Figures in parenthesis are calculated figures) |
|---|---|---|---|---|
| 17 | yellow viscous liquid | 1690 | 416<br>414 | C 63.79 H 5.42 N 6.58<br>(63.68) (5.59) (6.75) |
| 18 | brown viscous liquid | 1650 | 301<br>299 | C 71.97 H 6.18 N 4.77<br>(72.11) (6.05) (4.67) |
| 19 | reddish brown viscous liquid | 1660 | 278 | C 72.87 H 6.25 N 4.46<br>(72.72) (6.42) (4.46) |
| 20 | yellow solid | 1680 | 336<br>334 | C 64.68 H 5.17 N 3.92<br>(64.68) (5.13) (4.19) |
| 21 | brown viscous liquid | 1680 | 329 | C 69.14 H 6.28 N 3.95<br>(69.19) (6.11) (4.25) |

(continued)

TABLE 1 (continued)

| Compound No. | $R^1$ | $R^2$ $R^3$ | $R^4$ | $R^5$ |
|---|---|---|---|---|
| 22 | 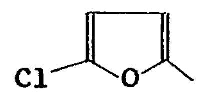 | Cl, H | 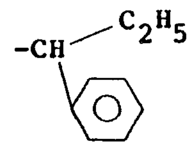 | $-CH_2OCH_3$ |
| 23 | 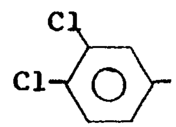 | Cl, H | 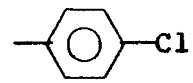 | 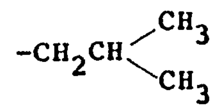 |
| 24 | 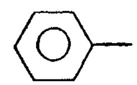 | Cl, H | 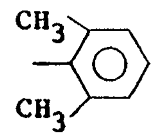 | $-CH_2Cl$ |
| 25 | 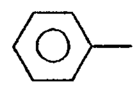 | Cl, H | 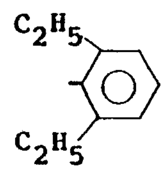 | $-CH_2Cl$ |
| 26 | 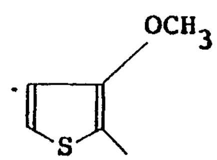 | Cl, H | 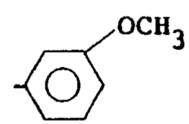 | $-CH_2CH_2F$ |

(continued)

TABLE 1 (continued)

| Compound No. | Appearance | IR($cm^{-1}$) (C=O) | Mass (m/e) | Elemental analysis (%) (Figures in parenthesis are calculated figures) |
|---|---|---|---|---|
| 22 | brown viscous liquid | 1650 | 349 | C 59.51 H 5.53 N 3.51<br>(59.70) (5.54) (3.66) |
| 23 | yellow solid | 1680 | 417<br>415 | C 54.55 H 4.26 N 3.44<br>(54.71) (4.11) (3.36) |
| 24 | yellow solid | 1685 | 336<br>334 | C 64.88 H 5.19 N 4.12<br>(64.68) (5.13) (4.19) |
| 25 | yellow viscous liquid | 1680 | 328 | C 66.31 H 5.80 N 3.92<br>(66.31) (5.84) (3.87) |
| 26 | brown viscous liquid | 1675 | 371<br>369 | C 55.41 H 4.81 N 3.60<br>(55.21) (4.63) (3.79) |

(continued)

TABLE 1 (continued)

| Compound No. | $R^1$ | $R^2$ $R^3$ | $R^4$ | $R^5$ |
|---|---|---|---|---|
| 27 | 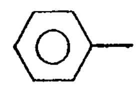 | Cl, $-CH_3$ | $-C_2H_5$ | $-CH_2OCH_3$ |
| 28 | 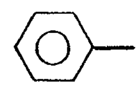 | Cl, $-CH_3$ | 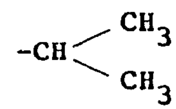 | $-CH_2Cl$ |
| 29 | 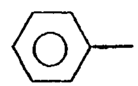 | Cl, $-CH_3$ | $-CH_2CH_2OCH_3$ | $-CH_2Cl$ |
| 30 | 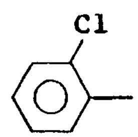 | Cl, $-CH_3$ | $-CH_2CH_2OCH_3$ | $-CH_2Cl$ |
| 31 | 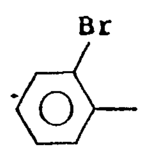 | Cl, $-CH_3$ | $-CH_2CH_2OCH_3$ | $-CH_2Cl$ |

(continued)

TABLE 1 (continued)

| Compound No. | Appearance | IR($cm^{-1}$) (C=0) | Mass (m/e) | Elemental analysis (%) (Figures in parenthesis are calculated figures) |
|---|---|---|---|---|
| 27 | yellow viscous liquid | 1680 | 270<br>368 | C 62.73 H 6.65 N 5.37<br>(62.80) (6.78) (5.23) |
| 28 | pale yellow viscous liquid | 1680 | 288<br>286 | C 58.99 H 6.10 N 4.76<br>(58.75) (5.99) (4.89) |
| 29 | pale yellow viscous liquid | 1670 | 268<br>266 | C 55.51 H 5.66 N 4.57<br>(55.64) (5.67) (4.63) |
| 30 | yellow viscous liquid | 1670 | 302<br>300 | C 50.04 H 4.84 N 4.36<br>(49.95) (4.79) (4.16) |
| 31 | yellow viscous liquid | 1680 | 345<br>343 | C 44.03 H 4.25 N 3.69<br>(44.12) (4.23) (3.68) |

(continued)

TABLE 1 (continued)

| Compound No. | $R^1$ | $R^2$ $R^3$ | $R^4$ | $R^5$ |
|---|---|---|---|---|
| 32 | 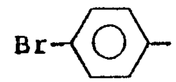 | Cl, $-CH_3$ | $-CH_2CH_2OCH_3$ | $-CH_2Cl$ |
| 33 | 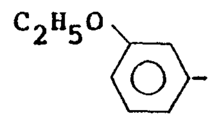 | Cl, $-CH_3$ | $-CH_2CH_2OCH_3$ | 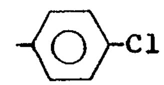 |
| 34 | 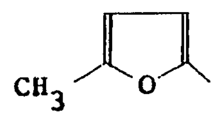 | Cl, $-CH_3$ | $-CH_2CH_2OCH_3$ | $-CH_2Cl$ |
| 35 | 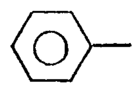 | Cl, $-CH_3$ | $-CH_2CH_2OC_2H_5$ | $-CH_2Cl$ |
| 36 | 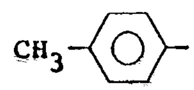 | Cl, $-CH_3$ | $-CH_2CH_2OC_2H_5$ | $-CH_2Cl$ |

(continued)

TABLE 1 (continued)

| Compound No. | Appearance | IR($cm^{-1}$) (C=0) | Mass (m/e) | Elemental analysis (%) (Figures in parenthesis are calculated figures) |
|---|---|---|---|---|
| 32 | pale yellow viscous liquid | 1680 | 345<br>343 | C 44.17 H 4.21 N 3.61<br>(33.12) (4.23) (3.68) |
| 33 | yellow solid | 1670 | 410<br>408 | C 61.73 H 5.59 N 3.40<br>(61.77) (5.68) (3.43) |
| 34 | yellow viscous liquid | 1670 | 308<br>306 | C 51.15 H 5.70 N 4.51<br>(51.00) (5.60) (4.57) |
| 35 | yellow viscous liquid | 1670 | 318<br>316 | C 56.81 H 6.06 N 4.38<br>(56.97) (6.06) (4.43) |
| 36 | yellow viscous liquid | 1670 | 296<br>294 | C 58.30 H6.74 N 4.40<br>(58.19) (6.41) (4.24) |

(continued)

TABLE 1 (continued)

| Compound No. | $R^1$ | $R^2$ $R^3$ | $R^4$ | $R^5$ |
|---|---|---|---|---|
| 37 | 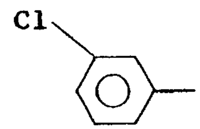 | Cl, $-CH_3$ | $-CH_2CH_2C_2H_5$ | $-CH_2Cl$ |
| 38 | 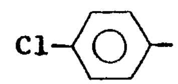 | Cl, $-CH_3$ | $-CH_2CH_2OC_2H_5$ | $-CH_2Cl$ |
| 39 | 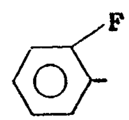 | Cl, $-CH_3$ | $-CH_2CH_2OC_2H_5$ | $-CH_2Cl$ |
| 40 | 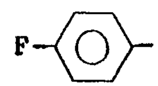 | Cl, $-CH_3$ | $-CH_2CH_2OC_2H_5$ | $-CH_2Cl$ |
| 41 | 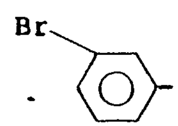 | Cl, $-CH_3$ | $-CH_2CH_2OC_2H_5$ | $-CH_2Cl$ |

(continued)

TABLE 1 (continued)

| Compound No. | Appearance | IR($cm^{-1}$) (C=O) | Mass (m/e) | Elemental analysis (%) (Figures in parenthesis are calculated figures) |
|---|---|---|---|---|
| 37 | yellow viscous liquid | 1670 | 351<br>350 | C 51.69 H 5.35 N 4.07<br>(51.38) (5.17) (3.99) |
| 38 | yellow viscous liquid | 1680 | 351<br>350 | C 51.52 H 5.32 N 4.14<br>(51.38) (5.17) (3.99) |
| 39 | yellow viscous liquid | 1670 | 335<br>334 | C 54.16 H 5.43 N 4.32<br>(53.91) (5.43) (4.19) |
| 40 | pale yellow viscous liquid | 1680 | 300<br>298 | C 54.06 H 5.47 N 4.34<br>(53.91) (5.43) (4.19) |
| 41 | yellow viscous liquid | 1670 | 359<br>357 | C 45.49 H 4.58 N 3.53<br>(45.60) (4.59) (3.54) |

(continued)

TABLE 1 (continued)

| Compound No. | $R^1$ | $R^2$ $R^3$ | $R^4$ | $R^5$ |
|---|---|---|---|---|
| 42 | 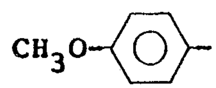 | Cl, $-CH_3$ | $-CH_2CH_2OC_2H_5$ | $-CH_2Cl$ |
| 43 | 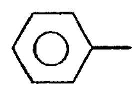 | Cl, $-CH_3$ | $-(CH_2)_3-OCH_3$ | $-CH_2Cl$ |
| 44 | 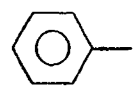 | Cl, $-CH_3$ | $-(CH_2)_3-OC_2H_5$ | $-CH_2Cl$ |
| 45 | 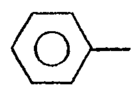 | Cl, $-CH_3$ | 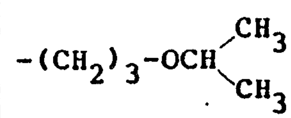 | $-CH_2CH_2Cl$ |
| 46 | 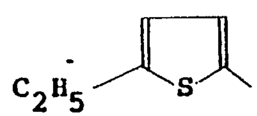 | Cl, $-CH_3$ | $-CH_2CH=CH_2$ | $-CH_2CH=CH_2$ |

(continued)

TABLE 1 (continued)

| Compound No. | Appearance | IR($cm^{-1}$) (C=0) | Mass (m/e) | Elemental analysis (%) (Figures in parenthesis are calculated figures) |
|---|---|---|---|---|
| 42 | yellow viscous liquid | 1670 | 347<br>345 | C 55.28 H 6.17 N 4.02<br>(55.50) (6.11) (4.05) |
| 43 | pale yellow viscous liquid | 1675 | 282<br>280 | C 56.99 H 6.06 N 4.48<br>(56.97) (6.06) (4.43) |
| 44 | yellow viscous liquid | 1670 | 296<br>294 | C 58.20 H 6.41 N 4.31<br>(58.19) (6.41) (4.24) |
| 45 | pale yellow viscous liquid | 1690 | 345<br>343 | C 59.13 H 6.72 N 4.26<br>(59.31) (6.73) (4.07) |
| 46 | yellow viscous liquid | 1670 | 312<br>310 | C 62.21 H 6.70 N 4.48<br>(62.02) (6.51) (4.52) |

(continued)

TABLE 1 (continued)

| Compound No. | $R^1$ | $R^2$ $R^3$ | $R^4$ | $R^5$ |
|---|---|---|---|---|
| 47 | 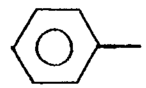 | Cl, $-CH_3$ | 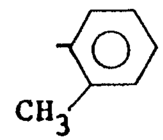 | $-CH_2Cl$ |
| 48 | 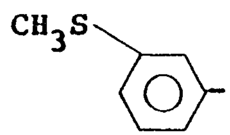 | Cl, $-CH_3$ | 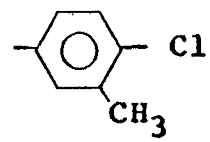 | $-CH_2OCH_3$ |
| 49 | 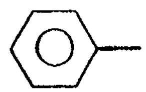 | Cl, $-C_2H_5$ | $-CH_2CH_2OC_2H_5$ | $-CH_2Cl$ |
| 50 | 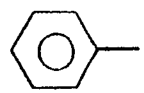 | Cl, $-C_2H_5$ | $-(CH_2)_3-SCH_3$ | 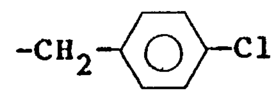 |
| 51 | 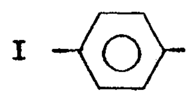 | Cl, $-C_2H_5$ | 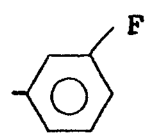 | 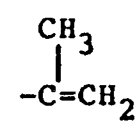 |

(continued)

TABLE 1 (continued)

| Compound No. | Appearance | IR($cm^{-1}$) (C=0) | Mass (m/e) | Elemental analysis (%) (Figures in parenthesis are calculated figures) |
|---|---|---|---|---|
| 47 | pale brown solid | 1690 | 300<br>298 | C 64.48 H 5.13 N 4.20<br>(64.68) (5.13) (4.19) |
| 48 | brown solid | 1685 | 412<br>410 | C 58.38 H 5.30 N 3.60<br>(58.54) (5.16) (3.41) |
| 49 | pale yellow viscous liquid | 1680 | 296<br>294 | C 58.21 H 6.47 N 4.27<br>(58.19) (6.41) (4.24) |
| 50 | yellow solid | 1670 | 424<br>422 | C 62.39 H 5.88 N 3.46<br>(62.56) (5.97) (3.32) |
| 51 | yellow viscous liquid | 1660 | 472<br>470 | C 51.00 H 3.92 N 3.10<br>(51.14) (3.86) (2.98) |

(continued)

TABLE 1 (continued)

| Compound No. | $R^1$ | $R^2$ $R^3$ | $R^4$ | $R^5$ |
|---|---|---|---|---|
| 52 | 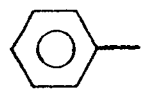 | Cl, $-C_2H_5$ | 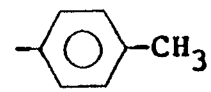 | $-CH_2Br$ |
| 53 | 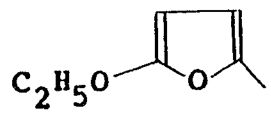 | Cl, $-C_3H_7$ | $-CH_2CH=CHCH_3$ | $-CHCl_2$ |
| 54 | 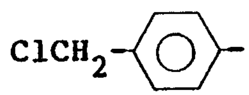 | Cl, $-C_3H_7$ | 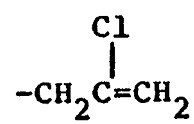 | $-C_2H_5$ |
| 55 | 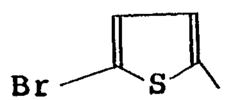 | Cl, $-isoC_3H_7$ | 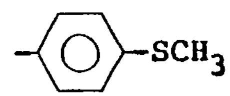 | $-CH_2CH_2OCH_3$ |
| 56 | 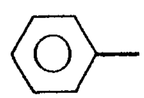 | Cl, $-C_4H_9$ | $-CH_2CH_2F$ | $-CH_2Cl$ |

(continued).

TABLE 1 (continued)

| Compound No. | Appearance | IR($cm^{-1}$) (C=0) | Mass (m/e) | Elemental analysis (%) (Figures in parenthesis are calculated figures) |
|---|---|---|---|---|
| 52 | brown viscous liquid | 1670 | 447<br>445 | C 51.22 H 3.49 N 3.25<br>(51.09) (3.61) (3.14) |
| 53 | yellow viscous liquid | 1670 | 358 | C 57.59 H 5.70 N 3.48<br>(51.73) (5.62) (3.55) |
| 54 | brown viscous liquid | 1675 | 376<br>374 | C 57.48 H 5.70 N 3.68<br>(57.69) (5.92) (3.74) |
| 55 | yellow viscous liquid | 1680 | 450<br>448 | C 49.30 H 4.80 N 2.69<br>(49.14) (4.74) (2.87) |
| 56 | pale yellow viscous liquid | 1675 | 296 | C 57.68 H 6.13 N 4.30<br>(57.84) (6.07) (4.22) |

(continued)

TABLE 1 (continued)

| Compound No. | $R^1$ | $R^2$ $R^3$ | $R^4$ | $R^5$ |
|---|---|---|---|---|
| 57 | 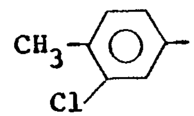 | Cl, $-C_4H_9$ | $-(CH_2)_3-CN$ | $-CH_2OCH_3$ |
| 58 | 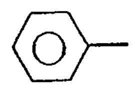 | Cl, $-C_5H_{11}$ | 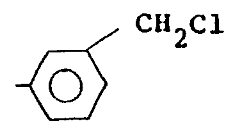 | $-CH_3$ |
| 59 | 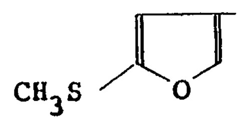 | Cl, $-C_6H_{13}$ | $-CH_2CH_2CH_3$ | $-CH=CH_2$ |
| 60 | 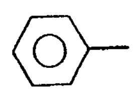 | Br, H | $-CH_3$ | 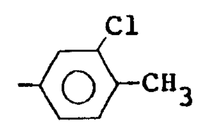 |
| 61 | 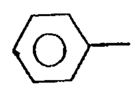 | Br, H | 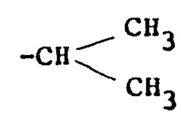 | $-CH_2OCH_3$ |

(continued)

TABLE 1 (continued)

| Compound No. | Appearance | IR($cm^{-1}$) (C=0) | Mass (m/e) | Elemental analysis (%) (Figures in parenthesis are calculated figures) |
|---|---|---|---|---|
| 57 | yellow viscous liquid | 1680 | 399<br>397 | C 60.37 H 6.61 N 7.11<br>(60.46) (6.60) (7.05) |
| 58 | yellow viscous liquid | 1670 | 392<br>390 | C 67.72 H 6.49 N 3.42<br>(67.69) (6.46) (3.59) |
| 59 | brown viscous liquid | 1670 | 372<br>370 | C 61.53 H 7.72 N 3.83<br>(61.69) (7.63) (3.79) |
| 60 | brown solid | 1660 | 365<br>363 | C 56.01 H 4.20 N 3.90<br>(55.99) (4.15) (3.84) |
| 61 | yellow viscous liquid | 1675 | 313<br>311 | C 53.70 H 5.76 N 4.52<br>(53.86) (5.81) (4.49) |

(continued)

TABLE 1 (continued)

| Compound No. | $R^1$ | $R^2$ $R^3$ | $R^4$ | $R^5$ |
|---|---|---|---|---|
| 62 | 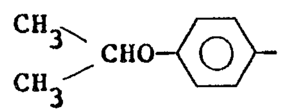 | Br, H | 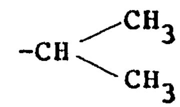 | $-CH_3$ |
| 63 | 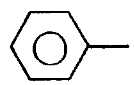 | Br, H | 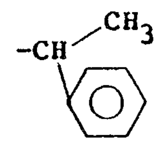 | $-CH_2Cl$ |
| 64 | 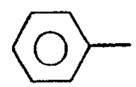 | Br, H | $-CH_2CH=CH_2$ | 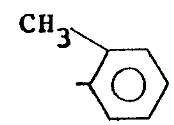 |
| 65 | 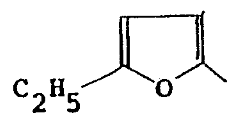 | Br, H | 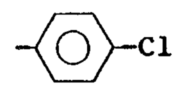 | $-CH_2SC_2H_5$ |
| 66 | 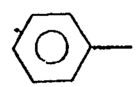 | Br, $-CH_3$ | $-C_2H_5$ | 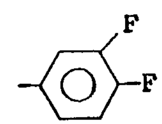 |

(continued)

TABLE 1 (continued)

| Compound No. | Appearance | IR($cm^{-1}$) (C=0) | Mass (m/e) | Elemental analysis (%) (Figures in parenthesis are calculated figures) |
|---|---|---|---|---|
| 62 | pale yellow viscous liquid | 1675 | 355<br>353 | C 57.61 H 6.77 N 3.82<br>(57.53) (6.83) (3.95) |
| 63 | yellow solid | 1660 | 381<br>379 | C 57.21 H 4.68 N 3.59<br>(57.09) (4.52) (3.70) |
| 64 | yellow solid | 1660 | 357<br>355 | C 64.22 H 5.16 N 3.78<br>(64.06) (5.09) (3.93) |
| 65 | brown viscous liquid | 1680 | 429<br>428 | C 50.36 H 4.53 N 3.40<br>(50.42) (4.47) (3.27) |
| 66 | pale yellow solid | 1670 | 381<br>379 | C 56.72 H 4.36 N 3.70<br>(56.86) (4.24) (3.68) |

(continued)

TABLE 1 (continued)

| Compound No. | Appearance | IR($cm^{-1}$) (C=0) | Mass (m/e) | Elemental analysis (%) (Figures in parenthesis are calculated figures) |
|---|---|---|---|---|
| 67 | pale yellow viscous liquid | 1670 | 362<br>360 | C 50.10 H 5.44 N 3.88<br>(49.95) (5.31) (3.88) |
| 68 | pale yellow viscous liquid | 1660 | 297<br>295 | C 45.41 H 4.40 N 3.74<br>(45.59) (4.59) (3.55) |
| 69 | pale yellow viscous liquid | 1665 | 355<br>354 | C 49.02 H 5.63 N 3.46<br>(49.18) (5.42) (3.59) |
| 70 | brown viscous liquid | 1660 | 386<br>384 | C 65.28 H 6.30 N 3.44<br>(65.46) (6.02) (3.64) |
| 71 | pale yellow viscous liquid | 1660 | 402<br>400 | C 61.11 H 6.40 N 3.38<br>(66.00) (6.55) (3.50) |

(continued)

TABLE 1 (continued)

| Compound No. | $R^1$ | $R^2$ $R^3$ | $R^4$ | $R^5$ |
|---|---|---|---|---|
| 72 | 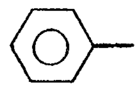 | Br, $-C_2H_5$ | $-CH_2CH_2Cl$ | $-CH_2CH_2CH_3$ |
| 73 | 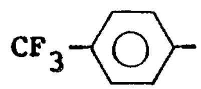 | Br, $-C_3H_7$ | $-CH_2CH=CH_2$ | $-CH_2OC_2H_5$ |
| 74 | 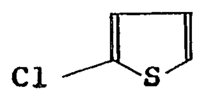 | Br, $-C_4H_9$ | $-(CH_2)_3-SC_2H_5$ | $-CH_2Cl$ |
| 75 | 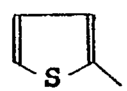 | Br, $-C_5H_{11}$ | 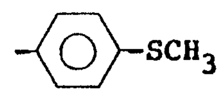 | $-CH_3$ |
| 76 | 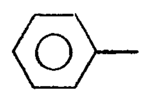 | F, H | $-(CH_2)_3-CH_3$ | $CH_3$<br>$-CH-C_3H_7$ |

(continued)

TABLE 1 (continued)

| Compound No. | $R^1$ | $R^2$ $R^3$ | $R^4$ | $R^5$ |
|---|---|---|---|---|
| 72 | 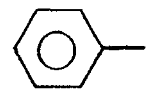 | Br, $-C_2H_5$ | $-CH_2CH_2Cl$ | $-CH_2CH_2CH_3$ |
| 73 | 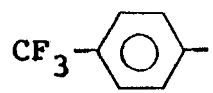 | Br, $-C_3H_7$ | $-CH_2CH=CH_2$ | $-CH_2OC_2H_5$ |
| 74 | 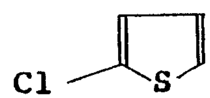 | Br, $-C_4H_9$ | $-(CH_2)_3-SC_2H_5$ | $-CH_2Cl$ |
| 75 | 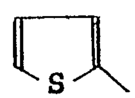 | Br, $-C_5H_{11}$ | 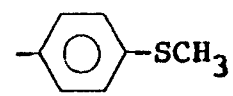 | $-CH_3$ |
| 76 | 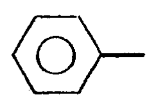 | F, H | $-(CH_2)_3-CH_3$ | 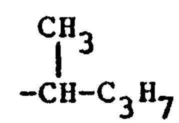 |

(continued)

TABLE 1 (continued)

| Compound No. | Appearance | IR($cm^{-1}$) (C=0) | Mass (m/e) | Elemental analysis (%) (Figures in parenthesis are calculated figures) |
|---|---|---|---|---|
| 72 | pale yellow viscous liquid | 1670 | 360<br>358 | C 53.61 H 5.77 N 3.77<br>(53.57) (5.90) (3.90) |
| 73 | yellow viscous liquid | 1675 | 436<br>434 | C 52.66 H 5.28 N 3.30<br>(52.55) (5.34) (3.23) |
| 74 | brown viscous liquid | 1665 | 443<br>441<br>439 | C 46.40 H 5.55 N 3.26<br>(46.27) (5.48) (3.17) |
| 75 | yellow viscous liquid | 1660 | 439<br>437 | C 54.60 H 5.40 N 3.40<br>(56.97) (5.52) (3.19) |
| 76 | pale yellow viscous liquid | 1675 | 291 | C 74.29 H 8.81 N 4.72<br>(74.19) (8.99) (4.81) |

(continued)

TABLE 1 (continued)

| Compound No. | $R^1$ | $R^2$ $R^3$ | $R^4$ | $R^5$ |
|---|---|---|---|---|
| 77 | 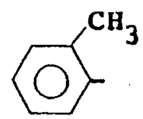 | F, $-CH_3$ | $-CH_2CH_2OCH_3$ | $-CH_2Cl$ |
| 78 | 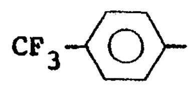 | F, $-CH_3$ | 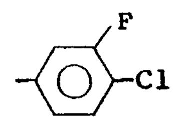 | $-C_2H_5$ |
| 79 | 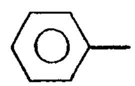 | I, H | $-(CH_2)_2-OC_3H_7$ | $-CH_2-$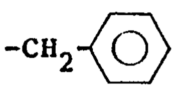 |
| 80 | 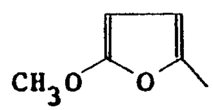 | I, $-CH_3$ | 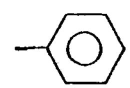 | $-CH_2-CH=CHCl$ |
| 81 | 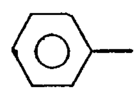 | I, $-C_2H_5$ | $-CH_2CH=CHCH_3$ | 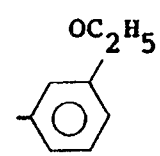 |

(continued)

TABLE 1 (continued)

| Compound No. | Appearance | IR($cm^{-1}$) (C=0) | Mass (m/e) | Elemental analysis (%) (Figures in parenthesis are calculated figures) |
|---|---|---|---|---|
| 77 | pale yellow viscous liquid | 1670 | 301<br>299 | C 60.30 H 6.11 N 4.50<br>(60.10) (6.39) (4.67) |
| 78 | yellow viscous liquid | 1660 | 384 | C 59.12 H 3.70 N 3.88<br>(59.30) (3.93) (3.64) |
| 79 | brown viscous liquid | 1670 | 450<br>448 | C 56.10 H 5.44 N 3.30<br>(56.13) (5.38) (3.12) |
| 80 | brown viscous liquid | 1660 | 459<br>457 | C 47.40 H 3.92 N 3.30<br>(47.23) (3.74) (3.06) |
| 81 | brown solid | 1665 | 477<br>475 | C 57.96 H 5.38 N 2.80<br>(58.11) (5.51) (2.95) |

(continued)

TABLE 1 (continued)

| Compound No. | $R^1$ | $R^2$ $R^3$ | $R^4$ | $R^5$ |
|---|---|---|---|---|
| 82 | 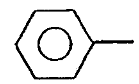 | Cl, Cl | 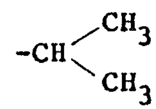 | 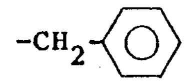 |
| 83 | 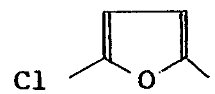 | Cl, Cl | $-CH_2CH_2CH_3$ | $-CH_2SCH_3$ |
| 84 | 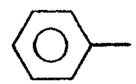 | Cl, Cl | $-CH_2CH_2OCH_3$ | $-CH_2Cl$ |
| 85 | 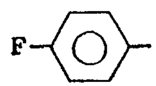 | Cl, Cl | $-CH_2CH_2OC_2H_5$ | $-CH_2OCH_3$ |
| 86 | 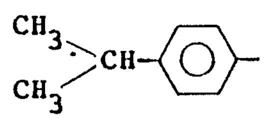 | Cl, Cl | 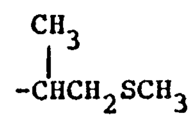 | $-CH_2Cl$ |

(continued)

TABLE 1 (continued)

| Compound No. | Appearance | IR($cm^{-1}$) (C=0) | Mass (m/e) | Elemental analysis (%) (Figures in parenthesis are calculated figures) |
|---|---|---|---|---|
| 82 | yellow solid | 1680 | 349<br>347 | C 65.38 H 5.61 N 4.13<br>(65.53) (5.50) (4.02) |
| 83 | yellow solid | 1660 | 342<br>340 | C 42.15 H 4.26 N 4.25<br>(42.06) (4.12) (4.09) |
| 84 | pale yellow solid | 1670 | 324<br>322 | C 48.50 H 4.34 N 4.29<br>(48.40) (4.37) (4.34) |
| 85 | pale yellow solid | 1670 | 351<br>349 | C 51.35 H 5.01 N 4.25<br>(51.44) (5.18) (4.00) |
| 86 | yellow solid | 1675 | 395<br>393 | C 51.39 H 5.76 N 3.38<br>(51.72) (5.62) (3.55) |

(continued)

TABLE 1 (continued)

| Compound No. | $R^1$ | $R^2$ $R^3$ | $R^4$ | $R^5$ |
|---|---|---|---|---|
| 87 | 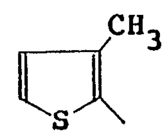 | Cl, Cl | $-(CH_2)_3-CN$ | 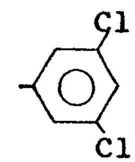 |
| 88 | 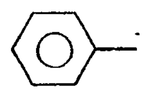 | Cl, Cl | $-CH_2CH=CH_2$ | $-CH_2Cl$ |
| 89 | 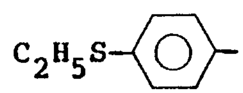 | Cl, Cl | $-CH_2CH=CCl_2$ | 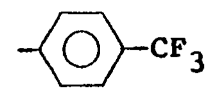 |
| 90 | 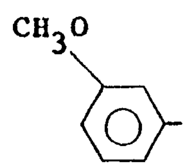 | Cl, Cl | 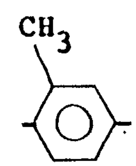 | $-C_2H_5$ |
| 91 | 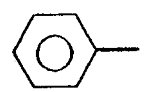 | Cl, Cl | 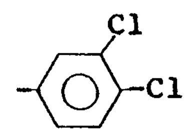 | 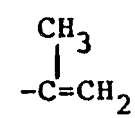 |

(continued)

TABLE 1 (continued)

| Compound No. | Appearance | IR($cm^{-1}$) (C=0) | Mass (m/e) | Elemental analysis (%) (Figures in parenthesis are calculated figures) |
|---|---|---|---|---|
| 87 | brown viscous liquid | 1665 | 448<br>446 | C 48.22 H 3.15 N 6.30<br>(48.24) (3.15) (6.25) |
| 88 | orange solid | 1680 | 304 | C 51.51 H 3.99 N 4.64<br>(51.26) (3.97) (4.60) |
| 89 | yellow solid | 1675 | 529<br>527 | C 47.50 H 3.11 N 2.55<br>(61.69) (3.05) (2.65) |
| 90 | pale yellow solid | 1680 | 365<br>363 | C 62.52 H 5.32 N 3.72<br>(62.65) (5.26) (3.85) |
| 91 | brown solid | 1675 | 401<br>399 | C 53.77 H 3.30 N 3.54<br>(53.90) (3.27) (3.49) |

(continued)

TABLE 1 (continued)

| Compound No. | $R^1$ | $R^2$ $R^3$ | $R^4$ | $R^5$ |
|---|---|---|---|---|
| 92 | 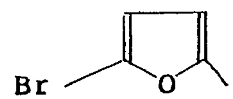 | Cl, Cl | 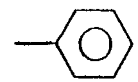 | $-CH_3$ |
| 93 | 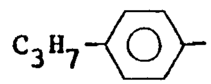 | Cl, Br | $-(CH_2)_3-CN$ | 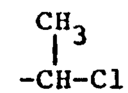 |
| 94 | 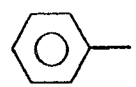 | Cl, Br | $-CH_2CH_2OCH_3$ | 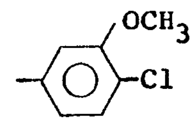 |
| 95 | 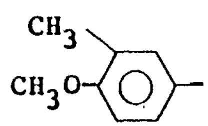 | Cl, Br | $-CH_2CH_2SCH_3$ | $-CH_2Cl$ |
| 96 | 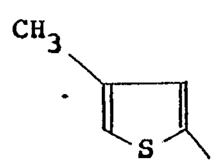 | Cl, Cl | 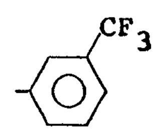 | $-CH_2OCH_3$ |

(continued)

TABLE 1 (continued)

| Compound No. | Appearance | IR($cm^{-1}$) (C=0) | Mass (m/e) | Elemental analysis (%) (Figures in parenthesis are calculated figures) |
|---|---|---|---|---|
| 92 | yellow solid | 1680 | 359<br>357 | C 46.70 H 2.95 N 3.76<br>(46.83) (2.81) (3.90) |
| 93 | brown solid | 1670 | 421<br>419 | C 51.11 H 5.75 N 3.21<br>(51.33) (5.74) (3.33) |
| 94 | yellow solid | 1660 | 459<br>457 | C 49.61 H 3.86 N 3.20<br>(49.70) (3.95) (3.05) |
| 95 | yellow solid | 1670 | 427<br>425 | C 42.32 H 4.41 N 3.40<br>(42.18) (4.25) (3.28) |
| 96 | yellow solid | 1670 | 470<br>468 | C 43.46 H 3.11 N 3.04<br>(43.56) (3.01) (2.99) |

(continued)

TABLE 1 (continued)

| Compound No. | $R^1$ | $R^2$ $R^3$ | $R^4$ | $R^5$ |
|---|---|---|---|---|
| 97 | 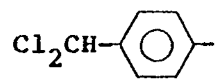 | Br, Br | $-C_2H_5$ | 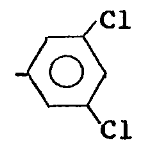 |
| 98 | 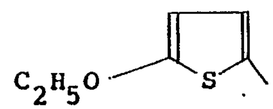 | Br, Br | $-CH_2CH_2CH_3$ | 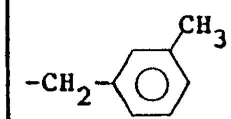 |
| 99 | 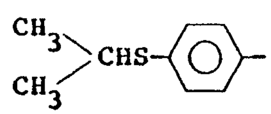 | Br, Br | $-CH_2CH_2CN$ | 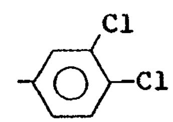 |
| 100 | 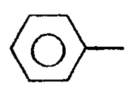 | Br, Br | 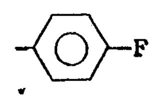 | $-CH=CHCH_3$ |
| 101 | 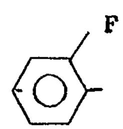 | F, F | $CH_3$<br>\|<br>$-CHCH_2OCH_3$ | 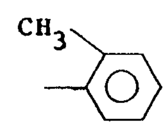 |

(continued)

TABLE 1 (continued)

| Compound No. | Appearance | IR($cm^{-1}$) (C=O) | Mass (m/e) | Elemental analysis (%) (Figures in parenthesis are calculated figures) |
|---|---|---|---|---|
| 97 | brown solid | 1675 | 559<br>557 | C 38.60 H 2.25 N 2.39<br>(38.54) (2.34) (2.50) |
| 98 | brown solid | 1670 | 489<br>487 | C 49.44 H 4.90 N 2.77<br>(49.51) (4.78) (2.89) |
| 99 | yellow solid | 1680 | 576<br>575 | C 43.79 H 3.29 N 4.83<br>(43.70) (3.14) (4.85) |
| 100 | yellow solid | 1670 | 439<br>437 | C 49.36 H 3.32 N 3.00<br>(49.23) (3.21) (3.19) |
| 101 | pale yellow solid | 1665 | 363 | C 65.99 H 5.36 N 3.77<br>(66.11) (5.55) (3.85) |

(continued)

TABLE 1 (continued)

| Compound No. | $R^1$ | $R^2$ $R^3$ | $R^4$ | $R^5$ |
|---|---|---|---|---|
| 102 | 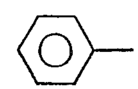 | I, I | $-CH_3$ | $-C_2H_5$ |
| 103 | 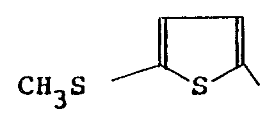 | I, I | $-CH_2CH=CH_2$ | $-CH_2Cl$ |
| 104 | 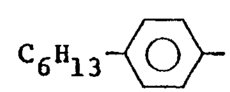 | Cl, H | 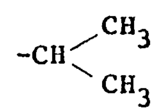 | $-CH_2Cl$ |
| 105 | 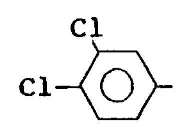 | Cl, $CH_3$ | $-C_2H_5$ | $-(CH_2)_5-CH_3$ |
| 106 | 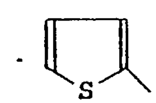 | Cl, $CH_3$ | 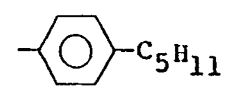 | $-CF_3$ |

(continued)

TABLE 1 (continued)

| Compound No. | Appearance | IR($cm^{-1}$) (C=0) | Mass (m/e) | Elemental analysis (%) (Figures in parenthesis are calculated figures) |
|---|---|---|---|---|
| 102 | brown solid | 1680 | 442<br>440 | C 32.69 H 2.69 N 3.33<br>(32.68) (2.97) (3.18) |
| 103 | brown solid | 1670 | 540<br>538 | C 26.49 H 2.40 N 2.75<br>(26.71) (2.24) (2.60) |
| 104 | yellow viscous liquid | 1660 | 357<br>355 | C 62.23 H 7.65 N 3.79<br>(64.04) (7.74) (3.93) |
| 105 | yellow viscous liquid | 1670 | 377<br>375 | C 57.50 H 6.33 N 3.86<br>(57.38) (6.42) (3.72) |
| 106 | brown solid | 1665 | 418<br>416 | C 57.39 H 5.08 N 3.44<br>(57.75) (5.09) (3.37) |

(continued)

TABLE 1 (continued)

| Compound No. | $R^1$ | $R^2$ $R^3$ | $R^4$ | $R^5$ |
|---|---|---|---|---|
| 107 | 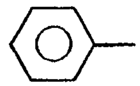 | Br, H | $-C_6H_{13}$ | $-CH_2Cl$ |
| 108 | 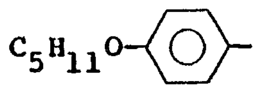 | Br, $CH_3$ | $-CH_2CH=CH_2$ | 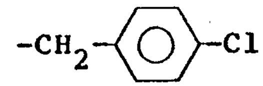 |
| 109 | 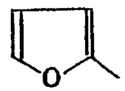 | Cl, H | 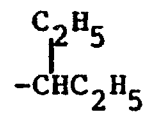 | $-CH_2OCH_3$ |
| 110 | 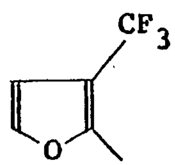 | Cl, H | $-(CH_2)_4-CH_3$ | $-CH_3CN$ |
| 111 | 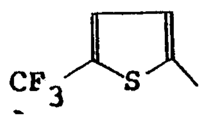 | Br, H | 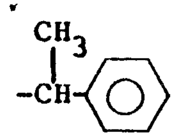 | $-C_2H_5$ |

(continued)

TABLE 1 (continued)

| Compound No. | Appearance | IR($cm^{-1}$) (C=0) | Mass (m/e) | Elemental analysis (%) (Figures in parenthesis are calculated figures) |
|---|---|---|---|---|
| 107 | brown viscous liquid | 1670 | 359<br>357 | C 53.60 H 5.77 N 3.83<br>(53.57) (5.90) (3.91) |
| 108 | yellow viscous liquid | 1680 | 491<br>489 | C 61.30 H 5.80 N 2.96<br>(61.17) (5.95) (2.85) |
| 109 | yellow viscous liquid | 1680 | 287<br>285 | C 58.94 H 7.12 N 4.75<br>(58.84) (7.05) (4.90) |
| 110 | pale brown viscous liquid | 1670 | 351<br>349 | C 51.72 H 4.92 N 8.11<br>(51.51) (4.90) (8.01) |
| 111 | yellow solid | 1690 | 417<br>415 | C 51.74 H 4.16 N 3.45<br>(51.93) (4.12) (3.37) |

(continued)

TABLE 1 (continued)

| Compound No. | $R^1$ | $R^2$ $R^3$ | $R^4$ | $R^5$ |
|---|---|---|---|---|
| 112 | H | Cl, H | 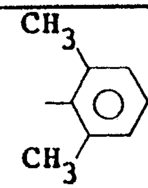 | $-CH_2Cl$ |
| 113 | H | H, Cl | 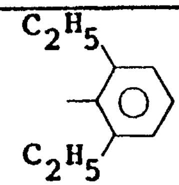 | $-CH_2Br$ |
| 114 | H | Cl, Cl | 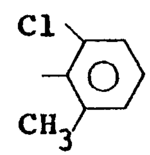 | $-CH_2Cl$ |
| 115 | H | Cl, Cl | 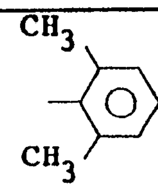 | 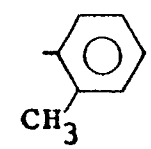 |
| 116 | 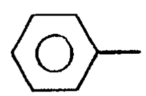 | Cl, Cl | 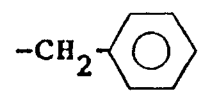 | $-CH_2Cl$ |

(continued)

TABLE 1 (continued)

| Compound No. | Appearance | IR($cm^{-1}$) (C=O) | Mass (m/e) | Elemental analysis (%) (Figures in parenthesis are calculated figures) |
|---|---|---|---|---|
| 112 | yellow solid | 1700 | 259<br>257 | C 55.69 H 5.12 N 5.40<br>(55.83) (5.08) (5.43) |
| 113 | yellow solid | 1700 | 331<br>329 | C 50.77 H 5.30 N 4.30<br>(50.86) (5.18) (4.24) |
| 114 | yellow viscous liquid | 1705 | 315<br>313 | C 42.37 H 3.16 N 4.50<br>(42.21) (2.90) (4.47) |
| 115 | yellow viscous liquid | 1680 | 300<br>298 | C 64.88 H 5.07 N 4.35<br>(64.68) (5.13) (4.19) |
| 116 | yellow solid | 1660 | 355<br>353 | C 57.40 H 4.03 N 3.86<br>(57.57) (3.98) (3.95) |

EXAMPLE 6

In an eggplant-shape flask were placed N-(2,2-dichloroethylidene)-2′,6′-dimethylaniline (3.00 g) and DMF (30 ml). Thereto was dropwise added a solution of chloroacetyl chloride (1.67 g) dissolved in DMF (5

ml), with stirring at room temperature. The mixture was stirred for 2 hours in an oil bath of 80 °C. The resulting mixture was washed with water, and the organic layer was extracted with ether. The extract was dried with sodium sulfate. The low-boiling materials were removed. The resulting liquid was subjected to distillation to obtain a yellow viscous liquid (2.46 g) having a boiling point of 124-125 °C/0.12 mmHg. The compound was measured for IR absorption spectrum. A strong absorption due to C=0 of amide was observed at 1700 $cm^{-1}$.

The compound was also measured for mass spectrum. There were seen characteristic peaks corresponding to $M^{\oplus}+1$, at m/e 294 and 292, and peaks corresponding $M^{\oplus}$ -Cl, at m/e 258 and 256.

The compound was further measured for $^1$H-NMR spectrum (δ, ppm: measured in deuterated chloroform using tetramethylsilane as a reference). The results of its analysis are as follows.

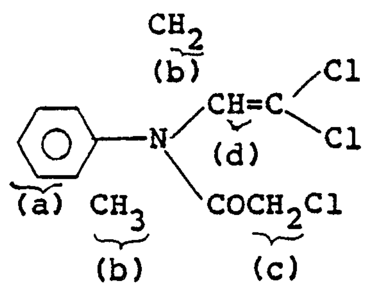

(a) 7.12 ppm (s, 3H)
(b) 2.21 ppm (s, 6H)
(c) 3.71 ppm (s, 2H)
(d) 7.57 ppm (s, 1H)

The elemental analysis was C 49.52 %, H 4.14 % and N 4.96 %. These figures agreed well with the calculated figures for $C_{12}H_{12}NCl_3O$ (292.60), i.e. C 49.26 %, H 4.13 % and N 4.79 %.

From the above results, it was confirmed that the isolated product was N-(2,2-dichloroethenyl)-N-chloroaceto-2′,6′-dimethylanilide. The yield was 61 %. This compound is designated as compound No. 117.

EXAMPLE 7

A nutrient medium containing 1.5 % of agar was sterilized at 121 °C for 15 minutes and then cooled to 50 °C. Thereto was added a suspension of microbial cells or spores in sterilized water, and the mixture was stirred thoroughly. The mixture was then poured into a laboratory dish and solidified thereon. A circular filter paper of 8 mm in diameter was immersed in a methanol solution containing about 15 % of the compound No. 1 synthesized in Example 1. An excess solution on the filter paper was removed, and the resulting filter paper was placed on the solidified agar medium. Culturing was effected for 24-96 hours at about 30 °C. Then, the diameter of the inhibition zone formed was measured.

The microbes used were as follows.

Batillus subtilis (natto Sawamura)
Cochliobolus miyabeanus
Trichophyton rubrum
Fusarium oxysporum

The results of the above antimicrobial test are shown in Table 2.

Table 2

| (Compound No. 1) | |
|---|---|
| Microbe | Diameter of inhibition zone (mm) |
| Batillus subtilis (natto Sawamura) | 11 |
| Cochliobolus miyabeanus | 20 |
| Trichophyton rubrum | 15 |
| Fusarium oxysporum | 11 |

EXAMPLE 8

The same antimicrobial test as in Example 7 was effected using various compounds synthesized in Examples 2-6.

The compound Nos. of tested compounds and their antimicrobial activities obtained are shown in Table 3.

Incidentally, E, B, A, C, T and F in Table 3 are abbreviated symbols for the following microbes.

E: Escherichia coil B
B: Batillus subtilis
A: Aspergillus niger
C: Cochliobolus miyabeanus
T: Trichophyton rubrum
F: Fusarium oxysporum

TABLE 3

| Compound No. | Antimicrobial activity (Figures in parentheses indicate a diameter of inhibition zone.) |
|---|---|
| 2 | B(12)A(13)C(11)T(15) |
| 3 | E(46)B(15)A(13)C(94)F(22) |
| 4 | C(50) |
| 5 | B(15)T(18) |
| 6 | E(10)B(13)A(12)C(11)F(30) |
| 7 | B(12)C(12)T(12)F(20) |
| 8 | T(15)F(20) |
| 9 | E(11)B(12)C(15)F(20) |
| 10 | B(11)C(14)F(15) |
| 11 | E(10)B(10)A(15)B(30)T(15)F(20) |
| 12 | T(18) |
| 13 | B(11)A(13)C(13)T(12)F(18) |
| 14 | E(11)B(11)C(20)T(13)F(15) |
| 15 | T(12) |
| 16 | T(15)F(12) |
| 17 | C(15) |
| 18 | C(30)T(12)F(16) |
| 19 | T(12) |
| 20 | E(40)B(40)A(34)C(100)T(15)F(12) |
| 21 | T(13) |
| 22 | E(20)A(15)C(15)T(12) |

(continued)

TABLE 3 (continued)

| Compound No. | Antimicrobial activity (Figures in parentheses indicate a diameter of inhibition zone.) |
|---|---|
| 23 | T(15) |
| 24 | B(10)A(20)C(21) |
| 25 | C(18)T(11) |
| 26 | T(12) |
| 27 | C(15)T(11) |
| 28 | T(12) |
| 29 | T(13) |
| 30 | T(15) |
| 31 | T(12) |
| 32 | C(50)T(12) |
| 33 | B(11) |
| 34 | B(12)C(15)T(14) |
| 35 | T(13) |
| 36 | C(13) |
| 37 | T(12) |
| 38 | T(11) |
| 39 | F(12) |
| 40 | T(12) |
| 41 | T(13) |
| 42 | C(30)T(13) |
| 43 | T(12) |

(continued)

TABLE 3 (continued)

| Compound No. | Antimicrobial activity (Figures in parentheses indicate a diameter of inhibition zone.) |
|---|---|
| 44 | T(13) |
| 45 | T(15) |
| 46 | B(12)T(14)F(11) |
| 47 | T(14) |
| 48 | A(11)F(14) |
| 49 | C(60)F(15) |
| 50 | T(11) |
| 51 | T(12) |
| 52 | B(11)F(12) |
| 53 | T(13) |
| 54 | T(11) |
| 55 | T(12)F(15) |
| 56 | C(14)T(11)F(13) |
| 57 | B(12)C(13) |
| 58 | T(13) |
| 59 | T(11) |
| 60 | T(15) |
| 61 | E(12)B(20)C(25)T(15) |
| 62 | B(13)C(40)T(11)F(12) |
| 63 | E(11)B(20)C(35)T(15)F(12) |
| 64 | C(12)F(11) |

(continued)

TABLE 3 (continued)

| Compound No. | Antimicrobial activity (Figures in parentheses indicate a diameter of inhibition zone.) |
|---|---|
| 65 | C(15)T(16) |
| 66 | T(15) |
| 67 | B(18)C(16)T(15) |
| 68 | B(10)C(12)F(10) |
| 69 | B(11) |
| 70 | B(12)T(13) |
| 71 | T(15) |
| 72 | T(14) |
| 73 | E(13)T(11) |
| 74 | C(15)T(12)F(14) |
| 75 | T(12) |
| 76 | B(15)C(20)T(11) |
| 77 | T(15) |
| 78 | T(13) |
| 79 | B(11)C(13) |
| 80 | C(14)T(13) |
| 81 | T(12) |
| 82 | B(15)C(13) |
| 83 | T(11) |
| 84 | B(10) |
| 85 | T(14) |

(continued)

TABLE 3 (continued)

| Compound No. | Antimicrobial activity (Figures in parentheses indicate a diameter of inhibition zone.) |
|---|---|
| 86 | B(13)C(30)T(16) |
| 87 | T(13) |
| 88 | B(10)C(14)T(10) |
| 89 | T(11)F(12) |
| 90 | T(15) |
| 91 | B(15)C(11)F(14) |
| 92 | T(11) |
| 93 | T(14) |
| 94 | B(11) |
| 95 | B(12)T(14) |
| 96 | B(14)C(12)T(13) |
| 97 | C(12) |
| 98 | T(11) |
| 99 | T(14) |
| 100 | T(10) |
| 101 | B(11)T(14) |
| 102 | T(13) |
| 103 | B(14)C(11)F(13) |
| 104 | E(12)B(15)A(16)C(14)F(13) |
| 105 | C(11) |
| 106 | B(13)C(12) |

(continued)

TABLE 3 (continued)

| Compound No. | Antimicrobial activity (Figures in parentheses indicate a diameter of inhibition zone.) |
|---|---|
| 107 | A(16)T(15) |
| 108 | T(12) |
| 109 | E(15)B(16)A(12)T(15)C(20)F(19) |
| 110 | T(13) |
| 111 | E(11)B(18)T(14)F(20) |
| 112 | E(12)B(15)A(25)C(20)F(30) |
| 113 | E(11)B(12)A(13)C(15)F(13) |
| 114 | E(10)B(13)A(28)C(20)T(10)F(30) |
| 115 | A(25)T(12)F(20) |
| 116 | B(14)C(18) |
| 117 | B(11)A(10)C(10)T(15)F(20) |

COMPARATIVE EXAMPLE 1

A compound [N-(2,2-dichloroethenyl)-N-imidazolyl-carbonyl-2′,6′-dimethylanilide] having the following structure was subjected to the same antimicrobial test as in Example 7. The microbes used were the same as used in Example 7. The compound formed no inhibition zone to any microbe and exhibited no antimicrobial activity.

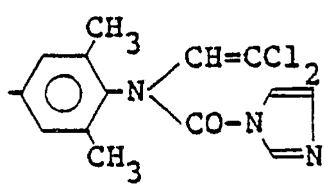

COMPARATIVE EXAMPLE 2

A compound [N-(1-phenylethenyl)-N-chloroacetobenzylamide] having the following structure was subjected to the same antimicrobial test as in Example 7. The microbes used were the same as used in Example 7. The compound formed no inhibition zone to any microbe and exhibited no antimicrobial activity.

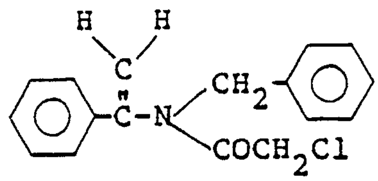

Preferable examples of the microbial agent containing the compound of the present invention are shown below.

| Preparation 1 (wettable powder) | |
|---|---|
| Compound No. 1 | 20 parts |
| Clay | 76 parts |
| Polyoxyethylene phenylalkylallyl ether sulfate (Na salt) | 2 parts |
| Sodium ligninsulfonate | 2 parts |

The above materials are mixed uniformly and then ground to obtain a wettable powder.

| Preparation 2 (emulsion) | |
|---|---|
| Compound No. 2 | 20 parts |
| Xylene | 70 parts |
| Polyoxyethylene phenylalkylallyl ether sulfate (Na salt) | 10 parts |

The above materials are mixed to obtain an emulsion.

| Preparation 3 (granules) | |
|---|---|
| Compound No. 3 | 10 parts |
| Bentonite | 30 parts |
| Talc | 55 parts |
| Dioctyl sulfosuccinate | 3 parts |
| Sodium tripolyphosphate | 2 parts |

The above materials are mixed throughly and then ground. Thereto is added water, and the mixture is stirred uniformly to form a paste. The paste is extruded from sieve meshes of 0.7 mm in diameter, dried and cut into a length of 1-2 mm to obtain granules.

| Preparation 4 (flowable) | |
|---|---|
| Compound No. 4 | 5 parts |
| 0.2 % aqueous xanthane rubber solution | 89 parts |
| Polyoxyethylene alkylallyl ether sulfate (Na salt) | 4 parts |
| Sodium ligninsulfonate | 2 parts |

The above materials are wet ground by a sand grinder to obtain a flowable.

## Claims

1. An amide compound represented by the following general formula (I)

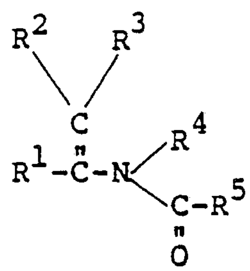

... (I)

wherein $R^1$ is a substituted or unsubstituted phenyl group, a substituted or unsubstituted furyl group, or a substituted or unsubstituted thienyl group; $R^2$ is a halogen atom; $R^3$ is a hydrogen atom, an alkyl group of 1-6 carbon atoms, or a halogen atom; $R^4$ and $R^5$ which may be the same or different, are a substituted or unsubstituted alkyl group of 1-12 carbon atoms, a substituted or unsubstituted alkenyl group of 2-12 carbon atoms, or a substituted or unsubstituted phenyl group; when $R^1$, $R^4$ and $R^5$ are each a substituted phenyl group or when $R^1$ is a substituted furyl group or a substituted thienyl group, the substituent(s) of each of the phenyl group, the furyl group and the thienyl group is (are) one to three members selected from the group consisting of halogen atoms, alkyl groups of 1-6 carbon atoms, alkoxy groups of 1-6 carbon atoms, alkylthio groups of 1-6 carbon atoms and halogenoalkyl groups of 1-4 carbon atoms; when $R^4$ and $R^5$ are each a substituted alkyl group or a substituted alkenyl group, the substituent(s) of each of the alkyl group and the alkenyl group is (are) one to three members selected from the group consisting of halogen atoms, alkoxy group of 1-6 carbon atoms, alkylthio groups of 1-6 carbon atoms, a cyano group and a phenyl group (this phenyl group may be substituted by one to three members selected from the group consisting of halogen atoms, alkyl groups of 1-6 carbon atoms, alkoxy groups of 1-6 carbon atoms and alkylthio groups of 1-6 carbon atoms).

2. An amide compound according to Claim 1, wherein in the general formula (I) $R^3$ is a hydrogen atom and $R^4$ is a branched alkyl group or a branched aralkyl group.

3. An amide compound according to Claim 2 wherein $R^4$ is a group of the formula

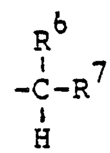

wherein $R^6$ is an alkyl group of 1 to 6 carbon atoms and $R^7$ is an alkyl group of 1 to 6 carbon atoms or a phenyl group.

4. An antimicrobial agent containing, as an active ingredient, an amide compound as claimed in Claim 1, 2 or 3.

5. An antimicrobial agent for use in agriculture, horticulture or industry, containing, as an active ingredient, an amide compound as claimed in claim 1, 2 or 3.

6. An antibacterial agent containing, as an active ingredient, an amide compound as claimed in Claim 1, 2 or 3.